# EUROPEAN PATENT APPLICATION

(11) **EP 2 140 876 A1**
(43) Date of publication of application: **06.01.2010**
(21) Application number: 08738932.6
(22) Date of filing: 26.03.2008
(51) Int. Cl.: A61K 38/19, A61K 48/00, A61P 13/12, A61P 43/00, C07K 14/435, C12N 15/12

(54) **THERAPEUTIC AGENT COMPRISING VASOHIBIN**

(30) Priority: 29.03.2007 JP 2007089122; 29.03.2007 JP 2007089123
(71) Applicant: National University Corporation Okayama University, Kita-ku Okayama-shi Okayama 700-8530 (JP); Shionogi & Co., Ltd., Osaka Osaka 541-0045 (JP)
(72) Inventor: SATO, Yasufumi, Sendai-shi, Miyagi 980-8577 (JP); MAESHIMA, Yohei, 5-1, Shikata-cho, 2-chome, Kita-ku Okayama-shi, Okayama 700-8558 (JP); NASU, Tatsuyo, 5-1, Shikata-cho, 2-chome, Kita-ku Okayama-shi, Okayama 700-8558 (JP); TANABE, Katsuyuki, 5-1, Shikata-cho, 2-chome, Kita-ku Okayama-shi, Okayama 700-8558 (JP); MAKINO, Hirofumi, 5-1, Shikata-cho, 2-chome, Kita-ku Okayama-shi, Okayama 700-8558 (JP)
(74) Representative: Stolzenburg, Friederike
(86) International application number: PCT/JP2008/055744
(87) International publication number: WO 2008/123308

(57) **Abstract**

A therapeutic agent containing Vasohibin for diabetic nephropathy, and a therapeutic agent containing Vasohibin for peritoneal sclerosis. Since the therapeutic agent containing Vasohibin of the present invention is a substance in which Vasohibin inhibits angiogenesis in an autocrine manner, the therapeutic agent is effective for inhibiting the progression of diabetic nephropathy and peritoneal sclerosis in which the inhibition in the angiogenesis or the production of cytokine is important, so that the therapeutic agent is suitably used in, for example, the treatment of a disease requiring an action for inhibiting the progression of diabetic nephropathy, and a disease requiring an inhibitory action for peritoneal sclerosis, or the like.

## Description

### TECHNICAL FIELD

The present invention relates to a Vasohibin-containing therapeutic agent. More specifically, the present invention relates to an inhibitor for progression in diabetic nephropathy, comprising Vasohibin, a therapeutic agent for diabetic nephropathy, containing the inhibitor, use of Vasohibin for the manufacture of a therapeutic agent for diabetic nephropathy, Vasohibin for use in the treatment of diabetic nephropathy, and a therapeutic method for diabetic nephropathy, including the step of administering the therapeutic agent. Also, the present invention relates to an inhibitor for peritoneal sclerosis comprising Vasohibin, a therapeutic agent for peritoneal sclerosis, containing the inhibitor, use of Vasohibin for the manufacture of a therapeutic agent for peritoneal sclerosis, Vasohibin for use in the treatment of peritoneal sclerosis, and a therapeutic method for peritoneal sclerosis, including the step of administering the therapeutic agent.

### BACKGROUND ART

Diabetic nephropathy is a disease which develops due to an increase in the level of albumin excreted in urine after the duration of morbidity of diabetes mellitus for a long time period, and the disease progresses to overt nephropathy showing persistent proteinuria, and further progresses to a chronic kidney disease such as chronic renal failure. A therapeutic method for the above-mentioned disease includes the control of blood pressure or blood glucose, and use of an ACE inhibitor or an angiotensin receptor antagonist. When the disease progresses to the end-stage renal failure disease, the therapeutic method includes primarily the dialysis therapy.

In addition, the diabetic nephropathy takes a hold on a first place in the leading primary disease of dialysis therapy in Japan, and the number introduced ever more increasingly every year. However, the dialysis therapy gives patients with a burden, so that a novel therapy is earnestly desired.

Non-Patent Publication 1 discloses erythropoietin as a promising agent for preventing the progression of renal failure. The publication reports that erythropoietin is an epoch-making agent which not only greatly decreases the necessity of blood infusions in patients undergoing dialysis and reduces a risk of hepatitis, or the like, but also remarkably improves the QOL of patients, thereby greatly changing the life of patients undergoing dialysis. In addition, Non-Patent Publications 2 and 3 describe therapeutic effects on the early stages of diabetic nephropathy by neutralizing anti-VEGF antibodies.

On the other hand, therapies for removing from blood a pathogenic substance, a toxic substance, or the like accumulated in the body are hemodialysis, peritoneal dialysis, hemodialysis filtration, blood adsorption, and the like. Among them, the peritoneal dialysis is the only therapeutic method which utilizes the peritoneal membrane as a semipermeable membrane (dialysis membrane) without subjecting to extracorporeal circulation, featuring in persistent dialysis.

However, if the peritoneal dialysis is continued for a long time period, the peritoneal membrane undergoes morphological and functional alterations. For example, Non-Patent Publication 4 reports that the peritoneal membranes of patients undergoing peritoneal dialysis have increases in thickness or cause fibrosis in the submesothelial compact zone, thereby causing changes in blood vessels. Non-Patent Publication 5 reports that if the peritoneal dialysis is performed over a long time period, the peritoneal mesothelial cells undergo a transition from an epithelial phenotype to a mesenchymal phenotype; and Non-Patent Publication 6 reports that the epithelial mesenchymal transition is involved in the peritoneal fibrosis induced by TGF-β overexpression. In addition, Non-Patent Publication 7 reports that expression of vascular endothelial growth factor VEGF is enhanced in the peritoneal membrane subjected to the peritoneal dialysis for a long time period. As described above, if the peritoneal dialysis is continued for a long time period, peritoneal sclerosis develops due to alterations in the peritoneal membrane, thereby making it difficult to continue the peritoneal dialysis.

On the other hand, Non-Patent Publication 8 discloses a therapeutic effect by a neutralizing anti-VEGF antibody, and Non-Patent Publication 9 discloses an inhibitory effect of the peritoneal fibrosis by an angiogenesis inhibitor TNP-470. However, there are no reports for treatment by agents other than these VEGF-associated molecules and TNP-470.
Non-Patent Publication 1: Jones M, Ibels L, Schenkel B, Zagari M, Kidney International, 2004, 65, 757-767
Non-Patent Publication 2: An S. De Vriese, et al., Journal of the American Society of Nephrology, 2001, 12, 993-1000
Non-Patent Publication 3: Allan Flyvbjerg, et al., Diabetes, 2002, 51, 3090-30941
Non-Patent Publication 4: John D. Williams, et al., Journal of the American Society of Nephrology, 2002, 13, 470-479
Non-Patent Publication 5: Maria Yanez-Mo, et al., The NEW ENGLAND JOURNAL of MEDICINE, 2005, 348, 403-413
Non-Patent Publication 6: Peter J. Margetts, et al., Journal of the American Society of Nephrology, 2005, 16, 425-436
Non-Patent Publication 7: Sophie Combet, et al., Journal of the Amerzcarr Society of Nephrology, 2000, 11, 717-728
Non-Patent Publication 8: Hiroaki Io, et al., Kidney International, 2004, 65, 1927-1936
Non-Patent Publication 9: Yoko Yoshio, et al., Kidney International., 2004, 66, 1677-1685

### DISCLOSURE OF THE INVENTION

### PROBLEMS TO BE SOLVED BY THE INVENTION

Although the diabetic nephropathy and the peritoneal sclerosis can be treated according to these conventional techniques, the mechanism for developing the above-mentioned disease has not been completely elucidated, so that a development of a novel therapeutic method is desired.

The present invention relates to a further useful substance having an action for inhibiting the progression of diabetic nephropathy, a therapeutic agent for diabetic nephropathy, containing the substance, use of the above-mentioned substance for the manufacture of a therapeutic agent for diabetic nephropathy, and a therapeutic method for diabetic nephropathy using the above-mentioned substance, and an inhibitor for peritoneal sclerosis comprising a substance other than the anti-VEGF antibodies or TNP-470, a therapeutic agent for peritoneal sclerosis, containing the inhibitor, use of the above-mentioned substance for the manufacture of a therapeutic agent for peritoneal sclerosis, and a therapeutic method for peritoneal sclerosis using the above-mentioned substance.

### MEANS TO SOLVE THE PROBLEMS

The present inventors have already clarified that Vasohibin acts as a negative feedback modulator in angiogenesis; however, an action of Vasohibin in diabetic nephropathy or peritoneal sclerosis has yet been remained unclarified. As a result of intensive studies, the present inventors have found that the progression of the diabetic nephropathy can be inhibited, or the peritoneal sclerosis can be inhibited, by using Vasohibin. The present invention has been completed thereby.

Concretely, the present invention relates to:
[1] a therapeutic agent for diabetic nephropathy, containing Vasohibin;
[2] a therapeutic agent for diabetic nephropathy, containing a vector containing a polynucleotide encoding Vasohibin;
[3] the therapeutic agent for diabetic nephropathy according to the above [2], wherein the vector is a viral vector;
[4] the therapeutic agent for diabetic nephropathy according to the above [3], wherein the viral vector is an adenoviral vector;
[5] use of Vasohibin for the manufacture of a therapeutic agent for diabetic nephropathy;
[6] use of a polynucleotide encoding Vasohibin for the manufacture of a therapeutic agent for diabetic nephropathy;
[7] Vasohibin for use in the treatment of diabetic nephropathy;
[8] a polynucleotide encoding Vasohibin for use in treatment of diabetic nephropathy;
[9] a therapeutic method for diabetic nephropathy, including the step of administering the therapeutic agent as defined in any one of the above [1] to [4];
[10] a therapeutic agent for peritoneal sclerosis, containing Vasohibin;
[11] a therapeutic agent for peritoneal sclerosis, containing a vector containing a polynucleotide encoding Vasohibin;
[12] the therapeutic agent for peritoneal sclerosis according to the above [11], wherein the vector is a viral vector;
[13] the therapeutic agent for the peritoneal sclerosis according to the above [12], wherein the viral vector is an adenoviral vector;
[14] use of Vasohihin for the manufacture of a therapeutic agent for peritoneal sclerosis;
[15] use of a polynucleotide encoding Vasohibin for the manufacture of a therapeutic agent for peritoneal sclerosis;
[16] Vasohibin for use in the treatment of peritoneal sclerosis;
[17] a polynucleotide encoding Vasohibin for use in treatment of peritoneal sclerosis; and
[18] a therapeutic method for peritoneal sclerosis, including the step of administering the therapeutic agent of as defined in any one of the above [10] to [13].

### EFFECTS OF THE INVENTION

According to the present invention, a further useful substance having an action for inhibiting the progression of diabetic nephropathy is provided. Also, according to the present invention, a further useful substance having an action for inhibiting the progression of peritoneal sclerosis is provided.

### BRIEF DESCRIPTION OF THE DRAWINGS

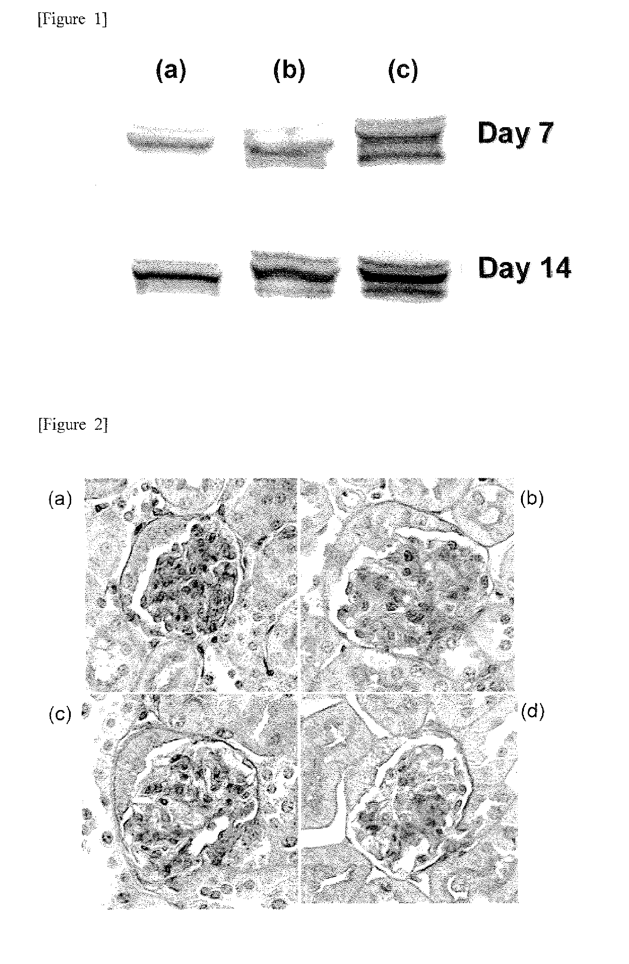

[Figure 1] Figure 1 shows the results of Western blotting of Vasohibin in serum, wherein (a) is the results of Western blotting of a serum from a physiological saline administration, (b) is those of a serum from AdVasohibin administration (1 × 10⁹ vp/mouse), and (c) is those of a serum from AdVasohibin administration (5 × 10⁹ vp/mouse).
[Figure 2] Figure 2 shows PAS-stained light microscopic sections of the kidney, wherein (a) is a section of a non-diabetic mouse group, (b) is that of a diabetic mouse-PBS administered group, (c) is that of a diabetic mouse-AdLacZ administered group, and (d) is that of a diabetic mouse-AdVasohibin administered group.

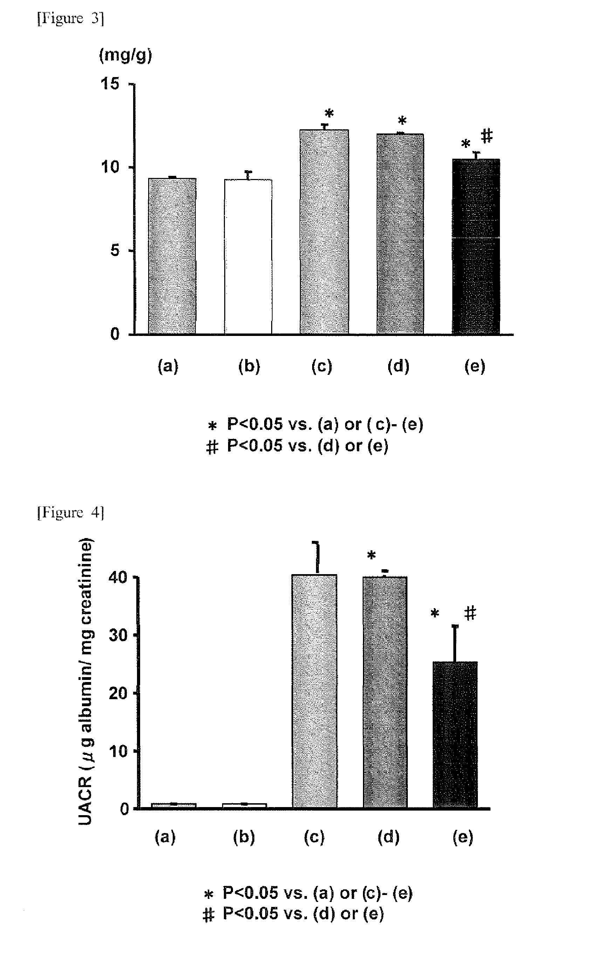

[Figure 3] Figure 3 is a graph showing a ratio of kidney weight to body weight (kidney weight/body weight ratio), wherein (a) is a non-diabetic mouse group, (b) is a non-diabetic mouse-AdVasohibin administered group, (c) is a diabetic mouse-PBS administered group, (d): diabetic mouse-AdLacZ administered group, and (e) is a diabetic mouse-AdVasohibin administered group.
[Figure 4] Figure 4 is a graph showing a ratio of albumin to creatinine in urine (urinal albumin/creatinine ratio, UACR), wherein (a) is a non-diabetic mouse group, (b) is a non-diabetic mouse-AdVasohibin administered group, (c) is a diabetic mouse-PBS administered group, (d) is a diabetic mouse-AdLacZ administered group, and (e) is a diabetic mouse-AdVasohibin administered group.

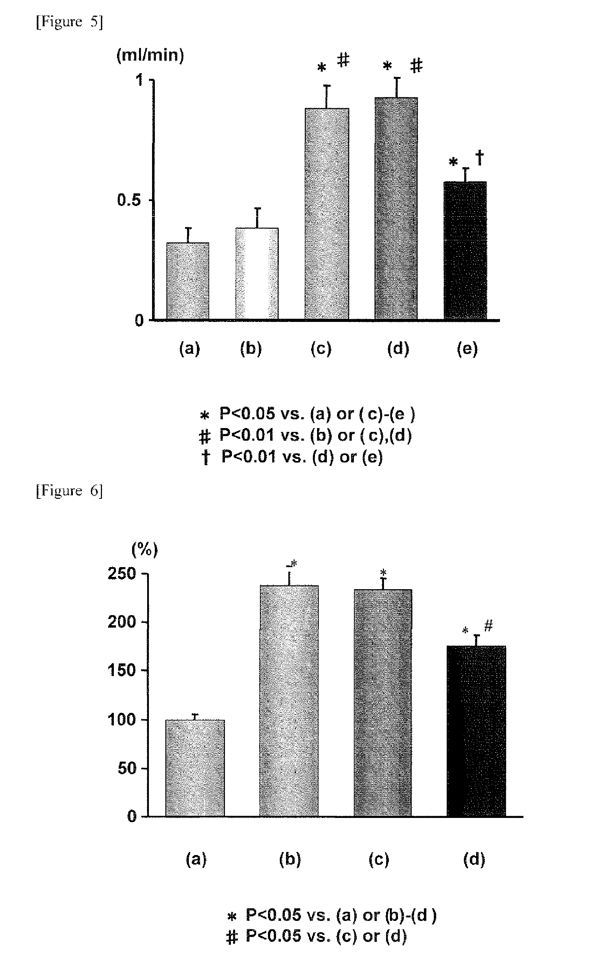

[Figure 5] Figure 5 is a graph showing a Creatinine clearance (Ccr) in 24 hours, wherein (a) is a non-diabetic mouse group, (b) is a non-diabetic mouse-AdVasohibin administered group, (c) is a diabetic mouse-PBS administered group, (d) is a diabetic mouse-AdLacZ administered group, and (e) is a diabetic mouse-AdVasohibin administered group.
[Figure 6] Figure 6 is a graph showing a volume ratio of glomeruli, wherein (a) is a non-diabetic mouse group, (b) is a diabetic mouse-PBS administered group, (c) is a diabetic mouse-AdLacZ administered group, and (d) is a diabetic mouse-AdVasohibin administered group.

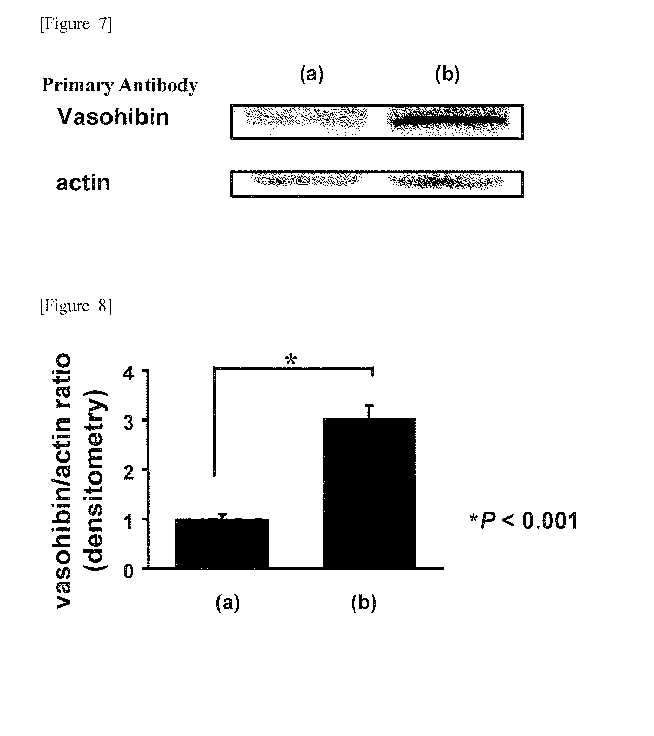

[Figure 7] Figure 7 shows the results of Western blotting of Vasohibin and actin in peritoneal tissues, wherein (a) is the results of Western blotting of a protein extracted from a group administered with a viral solution from a beta-galactosidase-expressing adenoviral vector, and (b) is those of a protein extracted from a group administered with purified viral solution from a human Vasohibin-expressing adenoviral vector.
[Figure 8] Figure 8 is a graph showing the results of densitometry analysis of Vasohibin and actin in peritoneal tissue, wherein (a) is a protein extracted from a group administered with a viral solution from a beta-galactosidase-expressing adenoviral vector, and (b) is a protein extracted from a group administered with a purified viral solution from a human Vasohibin-expressing adenoviral vector.

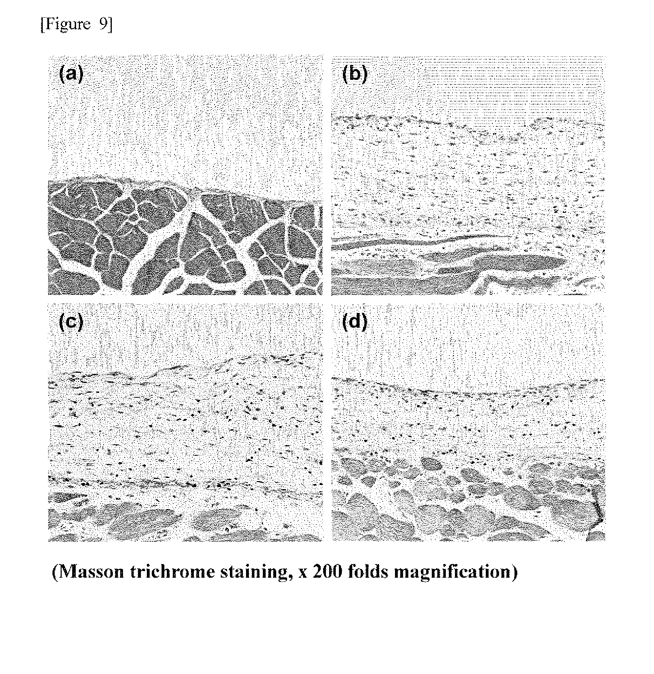

[Figure 9] Figure 9 shows PAS-stained light microscopic sections of peritonea, wherein (a) is a non-peritoneal sclerosis mouse group, (b) is a peritoneal sclerosis mouse group, (c) is a peritoneal sclerosis mouse-AdLacZ administered group, and (d) is a peritoneal sclerosis mouse-AdVasohibin administered group.

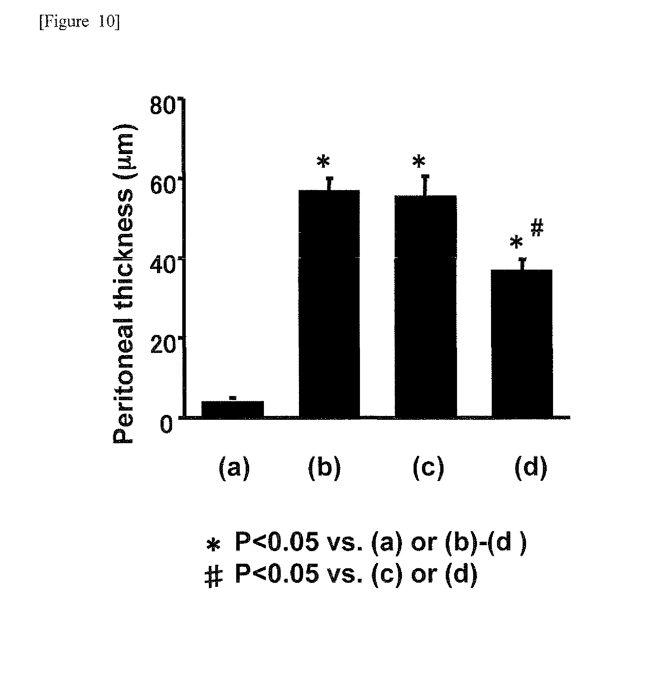

[Figure 10] Figure 10 is a graph showing a thickness (µm) of a thickened part of the peritoneal membrane, wherein (a) is a non-peritoneal sclerosis mouse group, (b) is a peritoneal sclerosis mouse group, (c) is a peritoneal sclerosis mouse-AdLacZ administered group, and (d) is a peritoneal sclerosis mouse-AdVasohibin administered group.

### BEST MODE FOR CARRYING OUT THE INTENTION

The present invention has a great feature in that Vasohibin is used in the inhibition of the progression of diabetic nephropathy. The inhibitor for the progression of diabetic nephropathy of the present invention is exemplified by an inhibitor comprising Vasohibin (embodiment 1), and an inhibitor comprising a vector containing a polynucleotide encoding Vasohibin (embodiment 2). Vasohibin is a substance for inhibiting angiogenesis that is expressed in vascular endothelial cells by a vascular endothelial growth factor (VEGF, FGF-2, and the like) secreted from a tumor cell, a mesenchymal cell, a macrophage, or the like, and acts in an autocrine manner to the endothelial cells themselves. Utilizing these excellent effects relating to the inhibition of angiogenesis possessed by Vasohibin, angiogenesis can be even more strongly inhibited by extracorporeally administering a Vasohibin protein or a Vasohibin gene. On the other hand, in the glomerular ansae of diabetic nephropathy, angiogenesis such as formation of new capillary vessels or extension of existing blood vessels takes place. In this process of angiogenesis, it is found that expression of a cytokine such as PDGF in activated endothelial cells is enhanced. In connection with this matter, a tissue alteration such as renal hypertrophy or glomerular hypertrophy takes place, thereby showing glomerular hyperfiltration, albuminuria, or the like, to develop diabetic nephropathy. In addition, it is known that monocyte/macrophage infiltration, an increase of expression of fibrosis promoting growth factor TGF-β or the like is involved in the process for the progression of diabetic nephropathy. Therefore, it is considered that the inhibition of angiogenesis and production of cytokine is important for inhibiting the progression of diabetic nephropathy. Since Vasohibin is a substance for inhibiting angiogenesis, it is deduced that diabetic nephropathy can be inhibited by extracorporeally administering a Vasohibin protein or a Vasohibin gene.

Also, the present invention has a great feature in the use of Vasohibin in the inhibition of the progression of peritoneal sclerosis. The inhibitor for peritoneal sclerosis of the present invention is exemplified by an inhibitor comprising Vasohibin (embodiment 1), and an inhibitor comprising a vector containing a polynucleotide encoding Vasohibin (embodiment 2), as mentioned above. In patients undergoing peritoneal dialysis, angiogenesis in the peritoneal membrane is increased, thereby causing the enhancement of peritoneal permeability. In addition, a positive correlation is found between an extent of thickness of peritoneal sclerosis and the number of blood vessels, so that it is known that the increase in the number of blood vessels has a possibility of promoting peritoneal sclerosis. However, there are some reports that a gene associated with angiogenesis is suppressed; as a result, the thickening of the peritoneal membrane is only somewhat ameliorated, even though the number of blood vessels is decreased and the peritoneal functions are recovered; therefore, a possibility of having some differences in the effects cannot be denied, depending upon by what means the angiogenesis is inhibited. As explained above, since Vasohibin is a substance that inhibits angiogenesis in an autocrine manner, it is deduced that peritoneal sclerosis can be inhibited in the form with even closer physiological phenomena by extracorporeally administering a Vasohibin protein or a Vasohibin gene.

Vasohibin in the present invention includes Vasohibin 1 and Vasohibin 2, and preferably includes Vasohibin 1. As disclosed in WO 02/090546, WO 2006/073052, and the like, Vasohibin 1 and Vasohibin 2 are different genes existing on different chromosomes, and the amino acid sequences of the proteins encoded by those genes have a 58% homology to each other, and both of the proteins have an inhibitory activity against angiogenesis. Vasohibin 1 refers to a protein encoded by KIAA1036 polynucleotide comprising a nucleotide sequence shown in A at position 386 to C at position 1480 of SEQ ID NO: 1, and KIAA1036 polypeptide comprising the amino acid sequence shown in SEQ ID NO: 2. Vasohibin 2 refers to a protein encoded by AY834202 polynucleotide comprising a nucleotide sequence shown in A at position 1 to G at position 1068 of SEQ ID NO: 3, and AY834202 polypeptide comprising amino acid sequence shown in SEQ ID NO: 4.

The polynucleotide in the present invention is exemplified by KIAA1036 polynucleotide comprising the nucleotide sequence shown in SEQ ID NO: 1, AY834202 polynucleotide comprising the nucleotide sequence shown in SEQ ID NO: 3, and a polynucleotide capable of hybridizing to the polynucleotide or a complementary strand thereto under stringent conditions.

The phrase "polynucleotide capable of hybridizing under stringent conditions" as used herein means a polynucleotide obtainable by using a well known conventional method in the art, such as a colony hybridization method, a plaque hybridization method, or a Southern blot hybridization method, using a fragment of the polynucleotide as a probe. Concretely, the polynucleotide means a polynucleotide which can be identified by carrying out hybridization at 65°C using a membrane immobilized with a polynucleotide derived from a colony or a plaque in the presence of 0.7 to 1.0 M NaCl, followed by washing the membrane at 65°C using 0.1 to 2-folds the concentration of SSC (saline sodium citrate: 150 mM sodium chloride, 15 mM sodium citrate). The hybridization can be carried out according to the methods described in the Molecular Cloning: A Laboratory Manual, Second Edition (1989) (Cold Spring Harbor Laboratory Press), Current Protocol in Molecular Biology (1994) (Wiley-Interscience), DNA Cloning 1: A Practical Approach Core Techniques, Second Edition (1995) (Oxford University Press), and the like. Here, a sequence composed only of adenine (A) or thymine (T) is preferably excluded from a sequence capable of hybridizing under stringent conditions.

The phrase polynucleotide capable of hybridizing" as used herein refers to a polynucleotide capable of hybridizing to another polynucleotide under the above-mentioned hybridization conditions. Concretely, the polynucleotide includes a polynucleotide which has a homology of 60% or more, preferably 80% or more, and more preferably 95% or more to KIAA1036 polynucleotide shown in SEQ ID NO: 1 and AY834202 polynucleotide shown in SEQ ID NO: 3. Incidentally, the homology as used herein is shown as a degree of resemblance in terms of a score by using the search program BLAST using an algorithm developed by Altschul et al. (The Journal of Molecular Biology, 215, 403-410 (1990)).

The above-mentioned polynucleotide can be prepared according to a known method, and the polynucleotide can be prepared, for example, according to a method disclosed in WO 02/090546. In addition, the polynucleotide can be prepared by chemically synthesizing a DNA encoding the KIAA1036 polypeptide or the AY834202 polypeptide on the basis of its amino acid sequence. The chemical synthesis of DNA can be carried out by using a DNA synthesizer manufactured by Shimadzu Corporation utilizing a thiophosphite method, a DNA synthesizer model 392 manufactured by PerkinElmer, Inc utilizing a phosphoamidite method, or the like.

The polypeptide in the present invention is exemplified by KIAA1036 polypeptide comprising the amino acid sequence shown in SEQ ID NO: 2, A834202 polypeptide comprising the amino acid sequence shown in SEQ ID NO: 4, a polypeptide having deletion, addition, insertion or substitution of one or several amino acid residues in the above-mentioned amino acid sequence, and a derivative thereof, and a salt thereof.

The phrase "a derivative (of a polypeptide)" as used herein refers to a derivative in which a polypeptide is subjected to acetylation, palmitoylation, myristylation, amidation, acrylation, dansylation, biotination, phosphorylation, succinylation, anilide formation, benzyl-oxycarbonylation, formylation, nitration, sulfonation, aldehydic formation, cyclization, glycosylation, monomethylation, dimethylation, trimethylation, guanidylation, amidination, maleylation, trifluoroacetylation, carbamylation, trinitrophenylation, nitrotroponylation, polyethylene-glycolation or acetoacetylation, or the like. Among them, an N-terminal acetylated derivative, a C-terminal amidated derivative, and a C-terminal methylated derivative are preferred, because the resulting polypeptide is provided with resistibility to exopeptidase which degrades a polypeptide from the terminal, so that it is expected that stability is enhanced in the living body even by glycosylation or polyethylene-glycolation.

The term "salt" as used herein refers to any pharmaceutically acceptable salt of polypeptides or derivatives thereof (including inorganic salts and organic salts). The salt includes, for example, sodium salts, potassium salts, calcium salts, magnesium salts, ammonium salts, hydrochlorides, sulfates, nitrates, phosphates, organic acid salts (acetates, citrates, maleates, malates, oxalates, lactates, succinates, fumarates, propionates, formates, benzoates, picrates, benzenesulfonates, and the like), and the like. Among them, it is desired that sodium salts, potassium salts and phosphates are preferred.

The above-mentioned polypeptide can be prepared according to a known method, and the polypeptide can be prepared, for example, according to a method disclosed in WO 02/090546, WO 2006/073052, or the like.

In addition, the above-mentioned derivative of the polypeptide can be prepared by a known method in the art. Also, the above-mentioned salt of the polypeptide can be easily prepared by a skill in the art by using any known method in the art.

The inhibitor of the embodiment 1 is substantially constituted by the above-mentioned Vasohibin, and the inhibitor of the embodiment 2 is constituted by a vector containing a polynucleotide encoding the above-mentioned Vasohibin.

It is preferable that the vector is autonomously replicable in a host cell, and at the same time is constructed by a promoter, a ribosome-binding sequence, a gene encoding Vasohibin, and a transcription termination sequence. In addition, the vector may contain a gene controlling a promoter. A preferred vector usable in the present invention includes vectors given later.

In addition, in the present invention, it is preferable that the vector is a viral vector, from the viewpoint of efficiency of expression in animal cells. The viral vector usable in the present invention is exemplified by a viral vector for infection of animal cells described later. Among them, an adenoviral vector is preferred. In the present invention, virus particles produced from a transformant harboring the vector are capable of infecting animal cells with the vector, whereby making it possible to express Vasohibin in an even higher efficiency in the animal cells. Therefore, the inhibitor for the progression of diabetic nephropathy and the inhibitor for peritoneal sclerosis of the present invention are also exemplified by an inhibitor comprising virus particles (embodiment 3) generated from the above-mentioned viral vector. Here, an inhibitor of a combination of an inhibitor comprising a viral vector further with virus particles generated from the viral vector, in other words, an inhibitor of a combination of the inhibitors of the embodiment 2 and the embodiment 3 is also embraced in the present invention.

The virus particles in the present invention are collected from a transformant and/or a culture medium by introducing the above-mentioned vector into various kinds of host cells, and culturing the cells according to an ordinary culture method suitable for those cells. The resulting collected product, that is, the virus particles may contain both of the culture medium and the transformant, and the virus particles can be purified and concentrated by a centrifugation method, with Adeno-X Virus Mini Purification kit (manufactured by Clontech), or Vivapure (manufactured by SARTORIUS).

Here, the above-mentioned vector, in other words, a vector containing a polynucleotide encoding Vasohibin, and a viral vector further containing viral DNA in addition to the polynucleotide can be prepared according to a known method, and the vector can be prepared, for example, according to a method disclosed in WO 02/090546, WO 2006/073052, or the like.

Since the inhibitor of the present invention can effectively inhibit kidney hypertrophy, glomerular hypertrophy, or the like in the diabetic nephropathy, the present invention further provides a therapeutic agent for diabetic nephropathy, containing the inhibitor of the present invention. Similarly, since the inhibitor of the present invention can effectively inhibit the thickening of the peritoneal membrane, or the like in the peritoneal sclerosis, the present invention further provides a therapeutic agent for a disease requiring an inhibitory action for peritoneal sclerosis, containing the inhibitor of the present invention.

In addition, with the progression, the diabetic nephropathy develops into renal failure or the like; since the inhibitor of the present invention can inhibit the progression of diabetic nephropathy, the present invention also provides a therapeutic agent for a disease requiring an action for inhibiting the progression of diabetic nephropathy, the therapeutic agent containing the inhibitor of the present invention. The disease requiring an action for inhibiting the progression of diabetic nephropathy for the treatment is not particularly limited, so long as the disease is a disease for which therapeutic effects are observed by inhibiting the progression of diabetic nephropathy, and the disease is exemplified by, for example, a chronic kidney disease. Since an effective therapy for inhibiting the progression of the disease has not yet been established for the chronic kidney disease, the application of the therapeutic agent of the present invention is to be expected.

Likewise, since the inhibitor of the present invention can effectively inhibit the thickening or the like of the peritoneal membrane in the peritoneal sclerosis, the present invention further provides a therapeutic agent for a disease requiring an inhibitory action for peritoneal sclerosis, the therapeutic agent containing the inhibitor of the present invention.

The disease requiring an inhibitory action for peritoneal sclerosis is not particularly limited, so long as the disease is a disease for which therapeutic effects are observed by inhibiting the progression of peritoneal sclerosis, and is exemplified by, for example, encapsulated peritoneal sclerosis. The causation of encapsulated peritoneal sclerosis is not only the peritoneal dialysis over a long time period, but an autoimmune disease, an intraperitoneal malignant tumor, a chemotherapy, an abdominal surgery, an infectious disease, or the like may also be the cause. Since there are no effective therapeutic methods in the encapsulated peritoneal sclerosis, the peritoneal dialysis must be stopped, so that the application of the therapeutic agent of the present invention is to be expected.

The therapeutic agent of the present invention includes one that is manufactured by combining the inhibitor of the present invention with a known pharmaceutical carrier to form into a formulation. In addition, in the therapeutic agent of the present invention, Vasohibin can be added together with other ingredients which are usable for the same application as Vasohibin, including, for example, a known ingredient having an action for inhibiting the progression of diabetic nephropathy, a known ingredient having action for inhibiting the progression of peritoneal sclerosis, or the like, such as a neutralizing anti-VEGF antibody.

The therapeutic agent of the present invention is usually manufactured by adding the inhibitor of the present invention with a pharmaceutically acceptable liquid or solid carrier, and a solvent, a dispersant, an emulsifier, a buffer, a stabilizer, an excipient, a binder, a disintegrant, a lubricant, or the like is added thereto as desired, so that a solid agent such as a tablet, a granule, a powder, a fine powder or a capsule, or a liquid agent such as a common liquid, a suspension or an emulsifier can be formed. Also, the therapeutic agent can be made into a dry product that can be made into a liquid state by adding an appropriate carrier prior to use, or other external preparations. Here, the pharmaceutical carrier is not particularly limited, and can be selected depending on the dosing form and the formulation form of the therapeutic agent.

The therapeutic agent in the form of the above-mentioned various formulations can be properly manufactured by a conventional method by utilizing each of known pharmaceutical carriers and the like. In addition, the amount of the inhibitor of the present invention contained in the therapeutic agent is not particularly limited, so long as the amount may be an amount so that the exhibition of the desired effects of the present invention can be obtained. The inhibitor of the present invention is contained in an amount of usually from 1 to 100% by weight or so of the therapeutic agent of the present invention.

The therapeutic agent of the present invention is administered by an appropriate method of administration depending upon the form of formulation. Also, the method of administration is not particularly limited. For example, the therapeutic agent can be administered internally, externally, or by an injection. In a case where the therapeutic agent of the present invention is administered by an injection, the therapeutic agent can be administered, for example, intravenously, intramuscularly, subcutaneously, intracutaneously, or the like. In a case where the therapeutic agent is administered externally, the therapeutic agent, for example, may be administered as an external formulation such as suppositories according to a suitable method of administration.

The dose of the therapeutic agent of the present invention is changeable and properly set depending on the form of the formulation, a method of administration, purpose of use and age, weight and symptoms of a patient to which the therapeutic agent is administered. In addition, the administration may be carried out in a single dose or several divided doses in a day within the desired dose range. The administration period is also arbitrarily determined.

The present invention also provides use of Vasohibin, and use of a polynucleotide encoding Vasohibin, for the manufacture of a therapeutic agent for diabetic nephropathy or a therapeutic agent for peritoneal sclerosis. Moreover, the present invention provides Vasohibin, and a polynucleotide encoding Vasohibin, for use in the treatment of diabetic nephropathy or peritoneal sclerosis.

The present invention also provides a therapeutic method for a disease requiring an action for inhibiting the progression of diabetic nephropathy, including the step of administrating Vasohibin to a subject. Similarly, the present invention provides a therapeutic method for a disease requiring an inhibitory action for peritoneal sclerosis, including the step of administrating Vasohibin to a subject.

The term "subject," as used herein preferably refers to human requiring an action for inhibiting the progression of diabetic nephropathy or an inhibitory action for peritoneal sclerosis, and the subject may also be a pet animal or the like.

In addition, the term "effective amount" as used herein refers to an amount of Vasohibin exhibiting an action for inhibiting the progression diabetic nephropathy, or an amount of Vasohibin exhibiting an inhibitory action for peritoneal sclerosis, when Vasohibin is administered to the above-mentioned subject, as compared to that of a subject not administered with Vasohibin. Concretely, the effective amount is changeable and properly set depending upon the form of administration, a method of administration, purpose of use, and age, weight and symptoms of a subject, and the like.

In the therapeutic method for a disease requiring an action for inhibiting the progression of diabetic nephropathy, and the therapeutic method for a disease requiring an inhibitory action for peritoneal sclerosis of the present invention, Vasohibin in an effective amount may be directly administered to the above-mentioned subject, or may be administered as a medicament such as a therapeutic agent as mentioned above. In addition, a method of administration is not particularly limited. For example, the administration may be carried out by an oral administration, an injection, or the like, in the same manner as in the medicament described above.

According to the therapeutic method of the present invention, a disease which would be an objective for the above-mentioned therapeutic agent of the present invention can be treated. For example, effects of treating a disease caused by diabetic nephropathy or peritoneal sclerosis as a causing factor can be exhibited.

In addition, the inhibitor for the progression diabetic nephropathy and the inhibitor for peritoneal sclerosis, each comprising a vector containing a polynucleotide encoding Vasohibin of the present invention can be used in gene therapy in a patient with a disease caused by diabetic nephropathy or peritoneal sclerosis as a causing factor.

The methods of introducing the inhibitor comprising a vector of the present invention to a patient are an *in vivo* method including the step of directly introducing the inhibitor into a body, and an *ex vivo* method including the steps of taking out a certain kind of a cell from human, introducing a DNA into the cell extracorporeally, and returning the resulting cell to the body [Nikkei Science, April, 20-45 (1994), Gekkan Yakuji (Monthly Pharmacology), 36, 23-48 (1994), Jikken Igaku (Experimental Medicine) supplement, 12, 15 (1994)]. In the present invention, the *in vivo* method is preferred.

When the inhibitor is administered by the *in vivo* method, the therapeutic agent is administered by a suitable administration pathway depending on diseases to be treated, targeted organs, and the like. For example, it is possible to directly locally administer the inhibitor to a tissue in which a lesion is found, or to administer the inhibitor intravenously, intraarterially, subcutaneously, intramuscularly, intraperitoneally, endoscopically, by aerosolization, or the like. The method of administration is preferably an intravenous or intraperitoneal administration. In addition, a direct injection to a tissue in which a lesion is found is preferred. For example, the tissue in which lesion is found is photographed by using any techniques utilizable in the art, such as nuclear magnetic resonance imaging or computed tomography, and the inhibitor comprising the vector of the present invention can be administered thereto by stereotactic injection.

In a case where the inhibitor comprising the vector of the present invention is used for a vector for gene therapy, as the form of the above-mentioned inhibitor, various forms of formulations suitable for each of the above-mentioned dosing form can be taken. For example, in a case where the inhibitor is used in the form of an injection containing a DNA encoding Vasohibin, which is an active ingredient, the injection can be prepared according to an ordinary method. The base used in the agent for gene therapy is not particularly limited, so long as the base is one usually used in injections. The base includes distilled water, sodium chloride, or salt solutions of mixtures of sodium chloride and inorganic salts or the like, solutions of mannitol, lactose, dextran, glucose or the like, amino acid solutions of glycine, arginine or the like, organic acid solutions, or mixed solutions of salt solutions and glucose solutions, and the like. In addition, according to an ordinary method, an injection may be prepared as a solution, a suspension or a dispersion using an auxiliary such as an osmotic adjustment agent, a pH adjustment agent, a vegetable oil such as sesame oil or soybean oil, or a surfactant such as lecithin or a nonionic surfactant in addition to these bases. These injections can be used in the form of a formulation that is readily dissolved upon use by a procedure such as powdering or lyophilization.

The amount of DNA encoding Vasohibin contained in the above-mentioned formulation varies depending upon a disease to be treated, a site to be administered, the number of administration, a period for desired treatment, age or weight of a patient, or the like, and can be properly adjusted. Usually, the amount of the DNA is generally from about 0.01 to 2000 mg, and preferably from 0.1 to 100 mg in terms of the weight of DNA encoding Vasohibin in a patient (calculated as 60 kg body weight).

The method for generating Vasohibin and the method for generating a vector for gene therapy are concretely described hereinbelow.

### (1) Method for Generating Vasohibin

Vasohibin can be generated by expressing a gene of Vasohibin in a host cell using the method described in Molecular Cloning: A Laboratory Manual, Second Edition. (1989) (Cold Spring Harbor Laboratory Press), Current Protocols in Molecular Biology (1994) (Wiley-Interscience), or the like, according to, for example, the following method.

A DNA fragment having a proper length, containing a part encoding a protein of Vasohibin is prepared as occasion demands, on the basis of a full length DNA encoding the protein. In addition, a DNA having a substitution of a base is prepared, so that a nucleotide sequence of a part encoding the protein is an optimal codon for expression in the host. The DNA is useful for improving the productivity of the protein. A recombinant DNA (a plasmid for expression) is generated by inserting the DNA fragment or a full length DNA into the downstream of a promoter of an appropriate expression vector. A transformant which produces Vasohibin can be obtained by introducing the plasmid for expression into a host cell compatible to the expression vector.

As a host cell, any of a prokaryotic cell, an animal cell, an insect cell, or the like can be used, so long as the cell can express a subject gene. As an expression vector, a vector which can autonomously replicate in the above-mentioned host cell or can be incorporated into chromosomes, and which contains a promoter at an appreciable position for the transcription of the gene encoding Vasohibin may be used.

### (i) A case where a prokaryote is used as a host

It is preferable that an expression vector of the Vasohibin protein can autonomously replicate in a prokaryote, and at the same time is constructed with a promoter, a ribosome-binding sequence, a gene encoding Vasohibin, and a transcription termination sequence. The vector may contain a gene controlling the promoter.

An expression vector can be exemplified by, for example, pBTrp2, pBTac1, pBTac2 (manufactured by Roche Diagnostics), Bluescript II SK(+), pBluescript II SK(-) (manufactured by STRATAGENE), pSTV28, pUCl18, pUC19 (manufactured by Takara Shuzo), pKK233-2 (manufactured by Amersham Bioscience), pSE280, pSupex, pUB110, pup5, pH194, pTrxFus (manufactured by Invitrogen), pGEMEX-1 (manufactured by Promega), pQE-8 (manufactured by QIAGEN), pGEX (manufactured by Pharmacia), pET system (manufactured by Novagen), pMAL-c2 (manufactured by New England Biolabs), pKYP10 (Japanese Patent Laid-Open No, Sho 58-110600), pKYP200 (Agricultural Biological Chemistry, 48, 669 (1984)), pLSA1 (Agricultural Biological Chemistry, 53, 277 (1989)), pGEL1 (Proceedings of the National Academy of Sciences URSA, 82, 4306 (1985)), pEG400 (Journal of Bacteriology, 172, 2392 (1990)), pTrs30 (FERM BP-5407), pTrs32 (FERM BP-5408), pGHA2 (FERM BP-400), pGKA2 (FERM B-6798), pA1 (Japanese Patent Laid-Open No. Sho 63-233798), pTerm2 (Japanese Patent Laid-Open No. Hei 3-22979, US4686191, US4939094, US5160735), or the like.

Any promoter which can express in a host cell such as *Escherichia coli* can be used. For example, the promoter includes a promoter from *Escherichia coli* or a phage, such as trp promoter (Ptrp), lac promoter (Plac), PL promoter, PR promoter, or PSE promoter; SPO1 promoter, SPO2 promoter, penP promoter, or the like. Also, an artificially modified promoter such as a promoter in which two Ptrps are connected in series (Ptrpx2), tac promoter, lac T7 promoter, or letI promoter can be used.

In addition, it is preferred that a plasmid in which a distance between Shine-Dalgano sequence which is a ribosome-binding sequence and an initiation codon is regulated to an appropriate distance of, for example, from 6 to 18 bases is used. A transcriptional termination sequence is not always needed for an expression of a gene of Vasohibin, but it is preferred that the transcription termination sequence is positioned at immediate downstream of the constructed gene.

The host cell includes, for example, a prokaryote of *Escherichia* genus, *Serratia* genus, *Bacillus* genus, *Brevibacterium* genus, *Corynebacterium* genus, *Microbacterium* genus, *Pseudomonas* genus, or the like. Examples are *Eschenichia* genus including XL1-Blue strain, XL2-Blue strain, DH1 strain, MC1000 strain, KY3276 strain, W1485 strain, JM109 strain, HB101 strain, No. 49 strain, W3110 strain, NY49 strain, BL21 (DE3) strain, BL21 (DE3) pLysS strain, HMS174 (DE3) strain and HMS174 (DE3) pLysS strain of *E. coli* or the like; *Serratia* genus including *S. ficaria* strain, *S. fonticola* strain, *S. liquefaciens, S. marcescens* strain or the like; *Bacillus* genus including *B*. *subtilis* strain, *B. amyloliquefacens* strain, or the like; *Brevibacterium* genus including *B. ammouiagenes* strain, *B. Immariophilum* (ATCC: 14068) strain, *B. saccharolyticum* (ATCC: 14066) strain, or the like; *Corynebacterium* genus including *C. glutamicum* (ATCC: 13032) strain, *C. glutamicum* (ATCC: 14067) strain, *C. gulutamicum* (ATCC: 13869) strain, *C. acetoacidophilum* (ATCC: 13870) strain, or the like; *Microbacterium* genus including *M. ammoniaphilum* (ATCC: 15354) strain, or the like, and *Pseudomonas* genus including *S. mephitica* strain, or the like.

Any methods for introducing an expression plasmid can be used, so long as the method is a method for introducing a DNA to the above-mentioned host cell. The method includes, for example, an electroporation method (Nucleic Acids Research, 16, 6127 (1988)), a calcium phosphate method (Proceedings of the National Academy of Scuences, USA, 69, 2110 (1972)), a protoplast method [Japanese Patent Laid-Open No. Sho 63-2483942; Gene, 17, 107 (1992), a method described in Molecular & General Genetics, 168, 111 (1979)], or the like.

### (ii) A case where an animal cell is used as a host

When an animal cell is used as a host, as an expression vector, for example, pcDNA1/Amp, pcDNA1, pCDM8, pREP4 (manufactured by Invitrogen), pHM6 (manufactured by Roche Diagnostics), pKK223-3, pGEX (manufactured by Amersham Biosciences), pAGE107 (Cytotechnology, 3, 133 (1990)), pAGE103 (The Journal of Biochemistry, 101, 1307 (1987)), pAmo, pAMoA (pAMoPRSA) (The Journal of Biological Chemistry, 268, 22782-22787 (1993)), pAS3-3(Japanese Patent Laid-Open No. Hei 2-22705), or the like can be used.

Any promoters can be used so long as the promoter can express the gene in a host. The promoter includes, for example, a promoter of IE (Immediate-early) gene of human cytomegalovirus (hCMV), an early promoter of SV40, a Long Terminal Repeat Promoter of Moloney Murine Leukemia Virus, a promoter of retrovirus, HSP promoter, SRα promoter, a promoter of metallothionein, or the like. Also, an enhancer of IE gene of hCMV can be used together with the promoter.

Examples of the animal cell to be used for a host are an established cell from human, such as HEK293 (human embryonic kidney cells, ATCC: CRL-1573), Namalwa (Burkitt lymphoma, ATCC: CRL-1432), HeLa (a cell of carcinoma of uterine cervix, ATCC: CCL-2), HBT5'637 (a leukemia cell, Japanese Patent Laid-Open No. Sho 63-299), BALL-1 (a leukemia cell) or HCT-15 (a large bowel cancer cell); an established cell from a mouse, such as Sp2/0-Ag14 (a mouse myeloma cell, ATCC: CRL-1581) or NSO (a mouse myeloma cell); an established cell from a monkey, such as COS-1 (African green monkey nephrocyte (SV40 transformed cell), ATCC: CRL-1650) or COS-7 (African green monkey nephrocyte (SV40 transformed cell), ATCC: CRL-1651), an established cell of from a hamster, such as CHO-K1 (Chinese hamster ovary cell, ATCC: ECL-61) or BHK-21 (C-13) (Sicilian hamster kidney cell, ATCC: CCL-10); an established cell from a rat, such as PC12 (an adrenal pheochromocytoma, ATCC: CRL-1721) or YB2/0 (a rat myeloma cell, ATCC: CRL-1662), or the like.

Any methods for introducing an expression plasmid can be used, so long as the method is a method for introducing a DNA into a host. The method includes, for example, an electroporation method (Cyto technology, 3, 133, (1990)), a calcium phosphate method (Japanese Patent Laid-Open No. Hei 2-22705), or a lipofection method (Proceedings of the National Academy of Sciences, USA, 84, 7413 (1987), Virology, 52, 456 (1973)). In addition, Vasohibin can be expressed by adding an expression vector containing a desired gene and a viral DNA for infection of an animal cell to a culture medium of an animal cell, and infecting the animal cell with a virus expressing the desired gene generated by recombination. A virus for infection includes an adenovirus, an adeno-associated virus, a retrovirus, or the like, and an adenovirus genome DNA-TPC (manufactured by TAKARA BIO INC.) can be preferably used as a viral DNA for infection.

### (iii) A case where an insect cell is used as a host

When an insect cell is used as a host, an expression vector includes, for example, pVL1392, pVL1393, pBlueBacIII, pFASTBac1 (manufactured by Invitrogen) or the like, and a virus for infection includes, for example, Baculovirus which infects an insect of *Maniestra brassicoe* family, Autographa california nuclear polyhedrosis virus (AcMNPV), Bac-N-Blue DNA, or the like. As a method for transforming an insect cell, a method described in Baculovirus Expression Vector: A Laboratory Manual (1992) (W. H. Freeman and Company*),* Molecular Cloning: A Laboratory Manual, Second Edition (1989) (Cold Spring Harbor Laboratory Press), Current Protocols in Molecular Biology (1994) (Wiley-Interscience), Biotechnology, 6, 47 (1988), or the like is used.

An expression vector containing a desired gene and a Baculovirus DNA for infection of an insect cell to a culture medium of an insect cell, and the insect cell is infected with a virus expressing the desired gene generated by recombination, whereby Vasohibin can be expressed.

An insect cell used for a host includes an established cell from *Spodoptera frugiperda,* an established cell from *Trichplusia ni,* or the like, and concrete examples are a cell from *S. frugiperda* including Sf9 (ATCC: CRL-1711, an ovary cell), Sf21 (an ovary cell), or the like; and a cell strain from .*T*. *ni* including High Five, BTI-TN-5B1-4 (an egg cell, manufactured by Invitrogen), or the like.

Any methods for introducing a plasmid for expression can be used, so long as the method is capable of introducing the plasmid into a host. The method includes, for example, a calcium phosphate method (Japanese Patent Laid-Open No. Hei 2-22705), a lipofection method (Proceedings of the National Academy of Sciences USA, 84, 7413 (1987)) or the like. In the lipofection method, CELLFECTIN reagent (Invitrogen) can be used. In addition, an electroporation method (Cytotechnology, 3, 133 (1990)), or the like, can be used in the same manner as in the animal cell.

### (iv) Method for Culturing Transformant

When a transformant harboring an expression plasmid incorporated with a DNA encoding Vasohibin is a cell such as *Escherichia coli* or an animal cell, cultivation is held according to usual culture method suitable for various kinds of hosts. The protein is produced and accumulated, and the protein is collected from a transformant or a culture medium, whereby the protein can be generated. When a transformant is an animal individual or a plant individual, the transformant is bred or cultivated according to a usual growth method suitable for various kinds of hosts. The protein is produced and accumulated, and the protein is collected from the animal individual or plant individual, whereby the protein can be generated.

When a host is an animal individual, for example a nonhuman transgenic animal carrying a gene encoding Vasohibin is bred, the Vasohibin encoded by the plasmid is produced and accumulated in the animal, and the protein is collected from the animal individual, whereby Vasohibin can be generated. A production and accumulation site of the protein in the animal individual includes, for example, milk, saliva, egg, or the like of the animal.

When a host is a prokaryote such as *Escherichia coli,* for example, a transformant carrying a gene encoding Vasohibin is cultured in a medium, Vasohibin encoded by the plasmid is produced and accumulated in the medium, and the protein is collected from the medium, whereby Vasohibin can be generated.

A method for culturing a transformant with Vasohibin can be carried out according to a usual method used in the culture of a host.

As a medium for culturing the resulting transformant, any of a natural medium or synthesized medium may be used, so long as the medium contains a carbon source, a nitrogen source, an inorganic salt or the like, which the organism can assimilate, and the medium is capable of efficiently culturing the transformant.

As a carbon source, any of carbon sources which each microorganism can assimilate, can be used. For example, carbohydrates such as glucose, fructose, sucrose, syrup containing them, starch or starch hydrolysate, an organic acid such as acetic acid or propionic acid, an alcohol such as ethanol or propanol, or the like can be used.

As a nitrogen source, an ammonium salt of various inorganic acids or organic acids such as ammonia, ammonium chloride, ammonium sulfate, ammonium acetate, or ammonium phosphate, other nitrogenous substances, peptone, meat extract, yeast extract, Corn Steep Liquor, casein hydrolysate, soybean cake and soybean cake hydrolysate, various kinds of fermentative bacteria or the digest, or the like can be used.

As an inorganic salt, potassium dihydrogenphosphate, potassium hydrogenphosphate, magnesium phosphate, magnesium sulfate, sodium chloride, ferrous sulfate, manganese sulfate, cupper sulfate, calcium carbonate, or the like can be used. Cultivation is held under an aerobic condition such as shaking or submerged culture.

When the transformant is a prokaryote such as *Escherichia coli,* as the medium, for example, an YT medium containing bactotryptone, an yeast extract and sodium chloride is preferred.

The incubation temperature is preferably from 15° to 40°C, and the culture time is usually from 5 hours to 7 days. During the cultivation, the pH is kept at 3.0 to 9.0. The adjustment of pH is carried out with an inorganic or organic acid, an alkali solution, urea, calcium carbonate, ammonia, or the like. In addition, an antibiotic such as ampicillin or tetracycline may be optionally added to the medium during the cultivation.

When a microorganism transformed with an expression vector using an inductive promoter as a promoter is cultured, an inducer may be optionally added to the medium. For example, when a transformant transformed with an expression vector using lac promoter is cultured, isopropyl-β-D-thiogalactopyranoside or the like may be added to the medium, and when a transformant transformed with expression vector using trp promoter is cultured, indole acrylic acid or the like may be added to the medium.

When a transformant for generating Vasohibin is an animal cell, as a medium for culturing the cell, RPMI1640 medium (The Journal of American Medical Association, 199, 519 (1967)), MEM medium (Science, 130, 432 (1959)), D-MEM medium (Virology, 8, 396 (1959)), that is generally used, or 199 medium (Proceedings of the Society for the Biological Medicine, 73, 1 (1950)), that is generally used, or a medium in which fetal calf serum (FCS) or the like is added to the medium, or the like can be used.

The cultivation is usually held under conditions of a pH of from 6 to 8 at 25° to 40°C in the presence of 5% CO₂, and the like, for 1 to 7 days. In addition, an antibiotic such as kanamycin, penicillin, or streptomycin may be optionally added to the medium during the cultivation.

When a transformant is an insect cell, as a medium for culturing the cell, TNM-FH medium (manufactured by Pharmingen), Sf-900II SFM medium (manufactured by Invitrogen), ExCell400, ExCell405 (manufactured by JRH Biosciences Inc.), Grace's Insect Medium (Nature, 195, 788 (1962)), or the like, that is generally used, can be used.

### (v) Generation Method

Vasohibin can be generated by culturing a transformant, and isolating and purifying Vasohibin from the culture medium. A method for isolating and purifying vasohibin can be carried out according to a well known conventional method in the art. As the method, for example, a method for isolating and purifying an enzyme or a method for purifying transglucosylase by Sandler et al. (Methods of Emzymology, 83, 458) can be used.

When Vasohibin is produced and accumulated as a soluble polypeptide, a culture medium in which a transformant is cultured as mentioned above is isolated to cells or fungal bodies and a medium by, for example, a method such as centrifugation. When Vasohibin exists in a host cell, cells or fungal bodies collected are washed with an appropriate buffer such as STE solution, and Vasohibin is then disrupted by ultrasonic waves, French press, Manton Gaulin homogenizer, Dynomill, or the like, and centrifuged or filtered, whereby a cell-free solution can be obtained.

A surfactant may be contained in the buffer used in the isolation and purification of Vasohibin in a proper amount. For example, sodium lauryl sulfate (SDS), N-lauroyisarcosine sodium (Sarcosyl), or the like may be contained.

A method of isolation and purification of a desired protein contained in the crudely purified product obtained can be carried out by a combination of various well-known methods of isolation and purification. Examples of the well-known methods include, for example, a solvent extraction method, a salting-out method with ammonium sulfate or the like, a dialysis method, a sedimentation method with an organic solvent, an ultrafiltration method, gel filtration, various kinds of chromatographic methods such as diethylaminoethyl (DEAE)-sepharose chromatography, anion chromatography or ion exchange chromatography using lysine such as DIAION HPA-75 (manufactured by Mitsubishi Chemical Corporation), cation chromatography using lysine such as S-Sepharose FF (manufactured by Pharmacia), hydrophobic chromatography using butyl sepharose or affinity chromatography, or various kinds of electrophoresis methods such as SDS-polyacrylamide gel electrophoresis method or isoelectric focusing electrophoresis method. The affinity chromatography can be carried out by using an antibody against Vasohibin.

When Vasohibin is produced and accumulated as an insoluble polypeptide, and cells or fungal bodies are isolated as mentioned above, and disrupted by an appropriate method in the same manner as above, and a fraction containing the polypeptide is then collected. A sample collected is solubilized with a solubilizer such as a surfactant such as sodium lauryl sulfate (SDS) or N-lauroylsarcosine sodium (Sarcosyl). The solubilized solution is diluted or dialyzed to a concentration that a solubilizer is not contained or hardly contained, and the polypeptide is constructed to a normal steric structure, and thereafter a purified preparation can be obtained by a method of isolation and purification in the same manner as mentioned above.

Also, Vasohibin can be produced as a fusion protein with other protein and purified by utilizing affinity chromatography with a substance having affinity with the fusion protein (YAMAKAWA, Akio, Jikken Igaku (Experimental Medicine), 13, 469-474 (1995)). Examples of an addition protein used as a fusion protein are protein A, FLAG, or the like (Proceedings of the Notional Academy of Sciences, USA, 86, 8227 (1989), Genes Development,4, 1288 (1990), Japanese Patent Laid-Open No. Hei 5-336963, or Japanese Patent Laid-Open No. Hei 6-823021). When a protein A is used, a fusion protein of Vasohibin and Protein A can be produced, and purified by affinity chromatography with an immunoglobulin G. When FLAG peptide is used, a fusion protein of Vasohibin and FLAG can be produced, and purified by affinity chromatography with an anti-FLAG antibody.

In addition to the above-mentioned method for generating Vasohibin with a transformant as mentioned above, Vasohibin can also be produced according to a known method using *in vitro* transcription/translation system (Journal of Biomolecular NMR, 6, 129-134 (1995), Science, 242, 1162-1164 (1998), or The Journal of Biochemistry, 110, 166-168 (1991)).

In addition, Vasohibin can be chemosynthesized on the basis of the amino acid sequence by a chemical synthesis method such as Fmoc method (Fluorenylmethyl oxycarbonyl method), tBoc method (t-butyl oxycarbonyl method), or a commercially available peptide synthesizing instrument, for example, APEX396 (manufactured by Advanced Chemtech), 433A (manufactured by Applied Biosystems), PS3 (manufactured by Protein Technologies), 9050 (manufactured by Perseptive) or PSSM-8 (manufactured by Shimadzu corporation).

### (2) Method for Generating Vector for Gene Therapy

A method of generating a vector for gene therapy, a method of expressing the vector in a cell, and the like are the same as those of the expression vector described in the above-mentioned "Method for Generating Vasohibin."

An expression vector is safe and low in toxicity, so that the expression vector can be administered to, for example, a mammal (for example, human, rat, mouse, rabbit, sheep, pig, cow, cat, dog, monkey, and the like). When an expression vector is used for gene therapy, it is preferable to use a DNA or RNA viral vector or plasmid vector capable of expressing the protein in the cell of a mammal including human having high safety. A viral vector preferred in gene therapy includes adenovirus, adeno-associated virus (AAV), retrovirus, poxvirus, herpes virus, herpes simplex virus, Lentivirus (HIV), Sendai virus, Epstein-Barr virus (EBV), vaccinia virus, poliovirus, Sindbis virus, SV40, or the like. A plasmid preferred in gene therapy includes pCAGGS (Gene, 108, 193-200(1991)), pBK-CMV, pcDNA3.1, pZeoSV (Invitrogen, Stratagene), or the like.

Here, by adding a signal sequence to a DNA encoding Vasohibin, the protein become a secretory protein, so that the protein is not necessarily locally administered. A protein produced and secreted in a cell acts to a distal target organ, thereby resulting in an action for inhibiting the progression of diabetic nephropathy. Therefore, it is possible to administer the protein to a normal tissue other than a pathological tissue or a normal cell. Here, when the protein is administered to human, an intravenous or intramuscular administration is preferred.

### EXAMPLES

The present invention will be more concretely described hereinbelow by the Examples, without intending to limit the scope of the present invention thereto. Here, unless specified otherwise, as a method of gene manipulation method, a method described in Molecular Cloning: A Laboratory Manual, 2nd Edition (Cold Spring Harbor Laboratory) is used.

### Example 1

### [Generation of Recombinant Adenovirus]

Plasmid pFLAG13-1036 (see WO 02/090546) was treated with restriction enzymes Notl and Xbal to collect a cDNA encoding human Vasohibin-1, and the cDNA encoding human Vasohibin-1 obtained was treated with T4DNA polymerase (manufactured by TAKARA BIO INC.) to make it blunt-ended, and purified. The purified cDNA was inserted into cosmid vector pAxCAwtit (manufactured by TAKARA BIO INC) digested with restriction enzyme Smil using Ligation Kit ver.2 (manufactured by TAKARA BIO INC.). The ligated product was treated with restriction enzyme Smil, and thereafter the fragment was subjected to packaging with Gigapack III XL Extract (manufactured by Stratagene). and *Escherichia coli* DH5α cells were infected therewith. The cells were spread over an agar plate containing ampicillin (manufactured by Sigma). thereby giving cosmid vector pAxCAwtit-Vh 1. The resulting vector was introduced into HEk293 cells together with adenovirus genome DNA-TPC (manufactured by TAKARA BIO INC.) using TransIT-293 reagent (manufactured by TAKARA BIO INC.), thereby giving a transformant producing a recombinant adenovirus (Ad-Vasohibin-1) by homologous recombination in the cells. The transfected 293 cells were cultured for 12 hours, and thereafter the cells were collected using EDTA/PBS(-). The resulting suspension was stepwise diluted, and the cells were spread again to a collagen-coated 96 well plate (manufactured by Corning), and then cultured for 18 days in a Dulbecco's modified Eagle's medium (DMEM, manufactured by Sigma) containing 10% fetal calf serum (FCS). The cells and the culture medium were collected from the well in which alteration of the cells was found, and the collected product was subjected to a procedure of freezing and thawing with dry ice and a water bath at 37°C that was repeated 6 times. The product obtained was centrifuged at 4°C and 5000 r/min for 5 minutes, the supernatant of which was stored as a primary viral solution at -80°C. Next, DMEM containing 10 µL of the above-mentioned primary viral solution and 5% FCS was added in an amount of 0.1 mL per well to 293 cells previously cultured to 70 to 100% confluent in a collagen-coated 24 well plate (manufactured by Corning), and the cells were infected with the virus. This procedure was carried out 4 times every 15 minutes, and 0.4 mL of DMEM containing 5% FCS thereto, and then the cells were collected from each of the culture media and subjected to a procedure of freezing and thawing that was repeated 6 times, and the product was centrifuged at 4°C and 5000 r/min for 5 minutes, the supernatant of which was stored as a secondary viral solution at -80°C. Here, beta-galatosidase expressing adenovirus AxCAiLacZ used as a negative control was purchased from TAKARA BIO INC.

### [Generation of Adenoviral solution Having High Titer]

A procedure of adding 0.5 mL of DMEM containing 15 µL of the resulting secondary viral solution and 5% FCS to the 293 cells previously cultured to 100% confluent in a 25 cm² collagen-coated flask (manufactured by Corning) was carried out 4 times every 15 minutes. Thereafter, 4.5 mL of DMEM containing 5% FCS was added thereto, and the cells were cultured for 3 days. Thereafter, the cells collected from each culture medium were disrupted with a tightly sealed sonicator, and centrifuged at 4°C and 3000 r/min for 10 minutes, and the collected supernatant was rapidly frozen with dry ice and stored at -80°C (a tertiary viral solution). Similarly, 293 cells previously cultured to 100% confluent in a 75 cm² collagen-coated flask (manufactured by Corning) were infected with 2 mL of DMFM containing 50 µL of this tertiary viral solution and 5% FCS, and 13 mL, of DMEM containing 5% FCS was added thereto. The cells were cultured for 3 days to prepare a viral solution similar to that of the tertiary viral solution (quaternary viral solution). The titer of the resulting virus was measured by TCID₅₀) method. Here, the quaternary viral solution was dispensed in an amount of I mL/vial, rapidly frozen with dry ice, and stored at -80°C until use.

### [Purification of Adenoviral solution]

About 1.0 pfu/mL of the quaternary viral solution was added to the 293 cells previously cultured to 70 to 100%confluent in six 225 cm² collagen-coated flasks, to infect the cells with the viral solution. This procedure was carried out 4 times every 15 minutes, DMEM containing 5% FCS were added thereto in an amount of 30 mL per flask, and the cells were cultured for 4 days. The cells collected from each culture medium were centrifuged at 4°C and 3000 r/min for 10 minutes, and 30 mL of supernatant were removed. Thereafter, the remaining cells were disrupted with a tightly closed sonicator to free the virus, and centrifuged at 4°C and 3000 r/min for 10 minutes to collect supernatant. Twenty milliliter of the viral solution was added to a tube overlaid with 10 mL of 4 M cesium chloride/10 mM HEPES, and 5 mL of 2.2 M cesium chloride/10 mM HEPES in this order, and the mixture solution were centrifuged at 4°C and 25,000 r/min for 2 hours with a swing rotor, and thereafter a virus band was collected with a syringe. Further, a saturated cesium chloride was added in an equivolume to the viral solution collected above in the syringe tube used in the above collection, and thereafter the syringe tube was overlaid with 2 mL of 4 M cesium chloride/10 mM HEPES and 4 mL of 2.2 M cesium chloride/10 mM HEPES in this order. The mixture was centrifuged at 4°C. and 35,000 r/min for 3 hours with a swing rotor, and a virus band was collected with a syringe. The collected viral solution was dialyzed overnight against 1 L of PBS (-) containing 10% glycerol, and the resulting purified viral solution was dispensed, and subjected to frozen storage at -80°C until use.

### Reference Example 1-1

In order to determine an optimal dose of a purified viral solution obtained, the following studies were made.

A purified viral solution from human Vasohibin expressing adenovirus vector obtained above was intravenously administered to the tail of five-week old male ICR mice (Streptozotocin-inducing type I diabetes model mice) at a dose of 1 × 10⁹ or 5 × 10⁹ vp/mouse. As a control, physiological saline was administered. (For each viral solution, each dose was administered to 3 mice each). On the seventh day and the fourteenth day from the administration, 3 mice each were sacrificed for each administration group, and blood was collected. Thereafter, serum was separated from the blood and stored at -20°C.

The serum sample obtained above was subjected to Western blotting. The Western blotting was carried out according to a known method (kidney International, 71, 227-238 (2006)). The expression level of Vasohibin in the serum sample was confirmed by ECL method (ECL solution, Amersham), using a monoclonal mouse anti-human Vasohibin antibody as a primary antibody. The results are shown in Figure 1. Here, in each lane, the sample was applied in an amount of 50 µg each.

From the above, it was found that an optimal dose of the purified viral solution was 5 × 10⁹ vp/mouse.

### Test Example 1-1

In order to examine whether the resulting purified viral solution has an action for inhibiting the progression of diabetic nephropathy, the following studies were made.

Streptozotocin (STZ: 120 mg/kg) was administered to five-week old male ICR mice three times for every 48 hours, thereby generating a diabetic nephropathy model mice (diabetic mice) in which diabetic nephropathy was induced. After having confirmed the high-blood glucose (250 mg/dL or more) in the resulting diabetic mice, PBS (300 µL/mouse, STZ-Ve group), AdLacZ (5 × 10⁹ vp/mouse, STZ-LacZ group) or AdVasohibin (5 × 10⁹ vp/mouse, STZ-Vas group) was intravenously administered to the tail of the above-mentioned mice (after one week from the STZ administration, n = 5 for each group). In addition, as controls, the group without administration (N group), and the group intravenously administering to the tail of AdVasohibin (5 × 10⁹ vp/mouse, Vas group) were furnished for ICR mice before the nephropathy was induced (non-diabetic mice) (n = 5 for each group). After two weeks, the group administered with AdLacZ or AdVasohibin was administered again with the same dose. Here, the blood glucose level and the blood pressure level were measured every week. Thereafter, the accumulated urine (24 hours) and the weight were measured at a point of five weeks from the administration of STZ, and these mice were then sacrificed. Thereafter, blood was collected, and the serum was then separated from the blood and stored at -20°C. Also, at the same time, the kidney was collected and weighed, thereafter the kidney was fixed with 10% formalin and embedded in paraffin, and paraffin sections having 4 µm in thickness were prepared.

PAS staining was performed for paraffin sections of an organ of each group. The results of the N group, the STZ-Ve group, the STZ-LacZ group and the STZ-Vas group are shown in Figure 2.

A ratio of albumin to creatinine in urine (urinal albumin/creatinine ratio, UACR), creatinine clearance (Ccr) in 24 hours and a ratio of kidney weight to body weight (kidney weight/body weight ratio) were measured. The results are shown in Figures 3 to 5. In addition, an area of glomeruli was measured by image analysis in PAS-stained light microscopic sections, and the volume of glomeruli was calculated therefrom. Each volume ratio of glomeruli was calculated, supposing that the area of glomeruli of the normal control group (N group) is 100%. The results are shown in Figure 6.

The diabetic mouse groups were found to have a significant high-blood glucose and weight loss compared to those of the non-diabetic mouse groups at a point of four weeks from the beginning of the treatment with administration of a viral solution, and influences by the administration with AdLacZ or AdVasohibin were not found between the diabetic mice groups. From the results of the kidney weight/body weight ratio, the diabetic mouse groups had significant hypertrophy of the kidney compared to those of the non-diabetic mouse groups, and among the diabetic mouse groups, the AdVasohibin administered group was found to have a significantly inhibitory effect for hypertrophy of the kidney as compared to that of the AdLacZ administered group. The diabetic mouse groups showed significantly high values for UACR and Ccr, as compared to those of the non-diabetic mouse groups, and among the diabetic mouse groups, the AdVasohibin administered group was found to have a significant inhibitory effect compared to the AdLacZ administered group. In view of the above, it is deduced that Vasohibin has a corrective effect of excess filtration by glomeruli in diabetic nephropathy. In addition, the volume of glomeruli significantly increased in the diabetic mouse groups as compared to that of the non-diabetic groups, and among the diabetic mouse groups, the AdVasohibin administered group was found to have an significant inhibitory effect as compared to that of the AdLacZ administered group. In view of the above, it is found that Vasohibin has an inhibitory effect for hypertrophy of glomeruli.

### Reference Example 2-1

Next, in order to examine whether the expression level of Vasohibin in the peritoneal membrane is enhanced by administration of the purified viral solution obtained, the following studies were made.

A purified viral solution from adenovirus vector expressing human Vasohibin (AdVasohibin) obtained above or a viral solution from adenovirus vector expressing beta-galactosidase (AdLacZ) as control was intraperitoneally administered to six-week old male ICR mice at a dose of 1 × 10⁹vp/mouse (500 µL) (each viral solutions were administered to three mice respectively). On the fourteenth day from the administration, these mice were sacrificed, and parietal peritoneal membrane was collected and stored at -80°C.

Protein extraction was carried out from the peritoneal tissues according to a conventional method, and thereafter the protein extract was subjected to Western blotting according to a known method (kidney International, 71, 227-238 (2006)). The expression level was confirmed according to ECL method (ECL solution: Amersham) using a monoclonal mouse anti-human Vasohibin antibody and a rabbit anti-actin antibody as primary antibodies. The densitometry analysis was carried out using an NIH image software, and Vasohibin/actin ratio was calculated for comparative study. The results of Western blotting are shown in Figure 7, and the results of densitometry analysis are shown in Figure 8. Here, in each lane of Western blotting, a sample was applied in an amount of 10 µg each.

In view of the above, it could be seen that the expression level of Vasohibin was enhanced by administration of the purified viral solution.

### Test Example 2-1

In order to examine whether the resulting purified viral solution has an action for inhibiting the progression of peritoneal sclerosis, the following studies were made.

Studies were made on the following 4 groups. 1) Normal groups (CG non-administered group, non-peritoneal sclerosis mouse), 2) CG-injected group, 3) CG-injected and AdLacZ-administered group, 4) CG-injected and AdVasohibin-administered group (n = 6 for each group)). Here, peritoneal sclerosis model mice (peritoneal sclerosis mice) were generated by intraperitoneally administering 0.1 % CG (chlorhexidine gluconate) to six-week old female ICR mice at a dose of (0/35 mL, every other day. In addition, AdVasohibin or AdLacZ used as a control was intraperitoneally administered on the day before the beginning of the administration with CG at a dose of 5 × 10⁹ vp/mouse. On the eighteenth day from the beginning of administration with CG, these mice were sacrificed, and parietal peritoneal membrane was collected. The collected peritoneal membrane was fixed with 10% formalin and embedded in paraffin, and paraffin sections having 4 µm in thickness were prepared. A thickening under mesothelial peritoneal membrane was calculated as a mean value (µm) of the thickness of the fibrous thickening region on a peritoneal muscle layer obtained by subjecting the mesothelial peritoneal membrane to Masson-trichrome staining, and observing the stained membrane at a magnification of 100 folds. The results are shown in Figures 9 and 10.

The peritoneal sclerosis mice develop infiltration of peritoneal inflammation cells and peritoneal fibrosis, as compared to the non-peritoneal sclerosis mice, and among the peritoneal sclerosis mouse groups, the AdVasohibin administered group had an inhibitory effect of infiltration of peritoneal inflammation cells and peritoneal fibrosis, as compared to the AdLacZ administered group. In addition, from the results of the thickening of the peritoneal membrane, the peritoneal sclerosis mouse groups were confirmed to have significant thickening of the peritoneal membrane as compared to the non-peritoneal sclerosis mouse groups, and among the peritoneal sclerosis mouse groups, the AdVasohibin administered group were found to have significant inhibitory effect of peritoneal sclerosis as compared to the AdLacZ administered group.

### INDUSTRIAL APPLICABILITY

The therapeutic agent containing Vasohibin of the present invention is suitably used in, for example, the treatment of a disease requiring an action for inhibiting the progression of diabetic nephropathy, and a disease requiring an inhibitory action for peritoneal sclerosis, or the like.

### SEQUENCE FREE TEXT

SEQ ID NO: 1: KIAA1036 polynucleotide.
SEQ ID NO: 2: KIAA1036 polypeptide.
SEQ ID NO: 3: AY834202 polynucleotide.
SEQ ID NO: 4: AY834202 polypeptide,

## Claims

1. A therapeutic agent for diabetic nephropathy, comprising Vasohibin.

2. A therapeutic agent for diabetic nephropathy, comprising a vector comprising a polynucleotide encoding Vasohibin..

3. The therapeutic agent for diabetic nephropathy according to claim 2, wherein the vector is a viral vector.

4. The therapeutic agent for diabetic nephropathy according to claim 3, wherein the viral vector is an adenoviral vector.

5. Use of Vasohibin for the manufacture of a therapeutic agent for diabetic nephropathy.

6. Use of a polynucleotide encoding Vasohibin for the manufacture of a therapeutic agent for diabetic nephropathy.

7. Vasohibin for use in treatment of diabetic nephropathy.

8. A polynucleotide encoding Vasohibin for use in treatment of diabetic nephropathy.

9. A therapeutic method for diabetic nephropathy, comprising the step of administering the therapeutic agent as defined in any one of claims 1 to 4.

10. A therapeutic agent for peritoneal sclerosis, comprising Vasohibin.

11. A therapeutic agent for peritoneal sclerosis, comprising a vector comprising a polynucleotide encoding Vasohibin.

12. The therapeutic agent for peritoneal sclerosis according to claim 11, wherein the vector is a viral vector.

13. The therapeutic agent for the peritoneal sclerosis according to claim 12, wherein the viral vector is an adenoviral vector.

14. Use of Vasohibin for the manufacture of a therapeutic agent for peritoneal sclerosis.

15. Use of a polynucleotide encoding Vasohibin for the manufacture of a therapeutic agent for peritoneal sclerosis.

16. Vasohibin for use in treatment of peritoneal sclerosis.

17. A polynucleotide encoding Vasohibin for use in treatment of peritoneal sclerosis.

18. A therapeutic method for peritoneal sclerosis, comprising the step of administering the therapeutic agent of any one as defined in claims 10 to 13.


SEQUENCE LISTING

<110> National University Corporation Okayama University
SHIONOGI&Co.,Ltd.

<120> Therapeutic agent comprising Vasohibin

<130> 08-011-PCTJP

<150> JP 2007-089122
<151> 2007-03-29

<150> JP 2007-089123
<151> 2007-03-29

<160> 4

<170> PatentIn version Ver. 2.1

<210> 1
<211> 5481
<212> DNA
<213> Homo sapiens

<220>
<221> CDS
<222> (386)..(1480)

<300>
<308> Genebank/XM#007415
<309> 2000-11-17

<400> 1
ccgctggtcc gagctgtctg gcctcagttt ccctccgact tttctccgct ctgccagccc 60

tcactgctgc ccgtcattgt tctcgcagtt agatgggggt gctttgtgac ggctgccaag 120

ttggggtgtg ttctctttat tccgtttttc aaacagaaca aggcctccaa ggctgacccc 180

agacaaccca ccccctcgga ccctaattca ccttattgca ctgatttttt ttatcaagtc 240

gtattttatt gtacaggagc cacgccctga tttcttaaag gcgccttgca ctctggccat 300

gtgttatctc tgcagccggt gtgtgggagg cctcttgtga gccagttgtt ttcccgcctc 360

caccaccccc ctcgaagatt taggg atg cca ggg ggg aag aag gtg gct ggg 412
Met Pro Gly Gly Lys Lys Val Ala Gly
1               5

ggt ggc agc agc ggt gcc act cca acg tcc gct gcg gcc acc gcc ccc 460
Gly Gly Ser Ser Gly Ala Thr Pro Thr Ser Ala Ala Ala Thr Ala Pro
10                  15                  20                  25

tct ggg gtc agg cgt ttg gag acc agc gaa gga acc tca gcc cag aga 508
Ser Gly Val Arg Arg Leu Glu Thr Ser Glu Gly Thr Ser Ala Gln Arg
                30                  35                  40

gat gag gag cca gaa gag gaa ggg gaa gag gac ctg cga gac gga ggc 556
Asp Glu Glu Pro Glu Glu Glu Gly Glu Glu Asp Leu Arg Asp Gly Gly
            45                  50                  55

gtc ccc ttc ttt gtc aac cgg ggt ggg cta cct gtg gat gag gcc acc 604
Val Pro Phe Phe Val Asn Arg Gly Gly Leu Pro Val Asp Glu Ala Thr
        60                  65                  70

tgg gaa agg atg tgg aaa cac gtg gcc aag atc cac ccc gat gga gag 652
Trp Glu Arg Met Trp Lys His Val Ala Lys Ile His Pro Asp Gly Glu
    75                  80                  85

aag gtg gcg caa cgg atc cgt ggg gcc aca gac ctg ccc aag atc ccc 700
Lys Val Ala Gln Arg Ile Arg Gly Ala Thr Asp Leu Pro Lys Ile Pro
90                  95                  100                 105

ata ccg agt gtg cct acg ttc cag ccg tct aca cct gtc cct gag cgc 748
Ile Pro Ser Val Pro Thr Phe Gln Pro Ser Thr Pro Val Pro Glu Arg
                110                 115                 120

ctg gaa gct gtg cag cgc tat atc aga gag ctg cag tac aat cac aca 796
Leu Glu Ala Val Gln Arg Tyr Ile Arg Glu Leu Gln Tyr Asn His Thr
            125                 130                 135

ggg aca cag ttc ttt gaa att aag aag agc aga cct ctg aca ggg ctg 844
Gly Thr Gln Phe Phe Glu Ile Lys Lys Ser Arg Pro Leu Thr Gly Leu
        140                 145                 150

atg gac ctg gcc aag gaa atg acc aaa gag gcc ctg cca atc aaa tgc 892
Met Asp Leu Ala Lys Glu Met Thr Lys Glu Ala Leu Pro Ile Lys Cys
    155                 160                 165

ctg gaa gcc gtg atc ctg gga att tac ctc acc aac agc atg ccc acc 940
Leu Glu Ala Val Ile Leu Gly Ile Tyr Leu Thr Asn Ser Met Pro Thr
170                 175                 180                 185

ctg gag cgc ttc ccc atc agc ttc aag acc tac ttc tca ggg aac tac 988
Leu Glu Arg Phe Pro Ile Ser Phe Lys Thr Tyr Phe Ser Gly Asn Tyr
                190                 195                 200

ttc cgc cac atc gtg ctg ggg gtg aac ttc gcg ggc cgc tac ggt gcg 1036
Phe Arg His Ile Val Leu Gly Val Asn Phe Ala Gly Arg Tyr Gly Ala
            205                 210                 215

ctg ggc atg agt cgg cgc gag gac ctg atg tac aag ccg ccc gcc ttc 1084
Leu Gly Met Ser Arg Arg Glu Asp Leu Met Tyr Lys Pro Pro Ala Phe
        220                 225                 230

cgc acg ctc agc gag ctc gtg ctg gac ttc gag gcc gcc tac ggc cgc 1132
Arg Thr Leu Ser Glu Leu Val Leu Asp Phe Glu Ala Ala Tyr Gly Arg
    235                 240                 245

tgc tgg cac gtg ctc aag aag gtg aag ctg ggc cag agc gtg tca cac 1180
Cys Trp His Val Leu Lys Lys Val Lys Leu Gly Gln Ser Val Ser His
250                 255                 260                 265

gac ccg cac agc gtg gag cag atc gag tgg aag cac tcg gtg ctg gac 1228
Asp Pro His Ser Val Glu Gln Ile Glu Trp Lys His Ser Val Leu Asp
                270                 275                 280

gtg gag cgc ctg ggc cgc gat gac ttc cgc aag gag ctg gag cgc cac 1276
Val Glu Arg Leu Gly Arg Asp Asp Phe Arg Lys Glu Leu Glu Arg His
            285                 290                 295

gcc cgc gac atg cgg ctc aag att ggc aaa ggg acg ggc cct ccc tct 1324
Ala Arg Asp Met Arg Leu Lys Ile Gly Lys Gly Thr Gly Pro Pro Ser
        300                 305                 310

ccc acc aag gac cgg aag aag gat gtt tct tcc ccg cag cgg gcc cag 1372
Pro Thr Lys Asp Arg Lys Lys Asp Val Ser Ser Pro Gln Arg Ala Gln
    315                 320                 325

tcc agc ccc cac cgc agg aac agc cgc agt gaa aga cgg ccc tcg ggt 1420
Ser Ser Pro His Arg Arg Asn Ser Arg Ser Glu Arg Arg Pro Ser Gly
330                 335                 340                 345

gac aag aag act tcc gag ccc aaa gcc atg cca gac ctt aac ggg tac 1468
Asp Lys Lys Thr Ser Glu Pro Lys Ala Met Pro Asp Leu Asn Gly Tyr
                350                 355                 360

cag atc cgg gtc tgaggcggat gccagcaccc caggccccac ccactcttgg 1520
Gln Ile Arg Val
            365

gggccaggat ccacctgctg gaaccagcct tatgcatggg gaaggcgggg ctggtgacaa 1580

ggcagggcaa gaggctgcag gaagagtgtg ttccagctca gccccccaag ctgctctcgc 1640

tcccactgag ccaagccccc taactttggg cctagaggcc gttagtattt tatttggagt 1700

ttttaactct acaactgaag tttaaggtat ttggggaaaa cttagtccaa atggatctgc 1760

tgatggtggg aaggccagtg cttaacaaat ccatgtgtca tggggccagg tgagggaaac 1820

tgctggttct gctggtgcct ctgcccctgg cttctctctg ggagttgggt gcatcttatc 1880

agtgggaaat ctcccagcct taccaggcct gtgatggggg gtgggggtgg ggtggagatg 1940

tttctccagt tctgcctgcc ctggcagaat cttgacccag ggaaagggaa gcagggtagg 2000

agtccttctg agaaaggtct gtgtagccca ttaaccagga gcttggcaca ggccacatct 2060

gccccaagag catgagctcg tggctcagga gagccttcag gcccttgagg cccccatggg 2120

cagtgctgtg tgggcagagg aggggtgata tgagagcgag cccagggaag gacctctggg 2180

caaaaagttc ccaggcccta actgcgtcta cttgctcagt cccagctctg cctgttgctc 2240

tagcccacag gctccctgcg gagggtctgg gcttggcgga ggacccagaa tggcactgag 2300

gccagcatgg ctgtgggagt tgagcaaacc ctgggtgcca gtccaggagc tgtggctgga 2360

catggggtga tggggcggga tgcttgggcc tgggtctctc cgccagcagt gccaggagcc 2420

cttgctgggg aaatcaagac cagactagga tgcttctgcc ccaggcctgc cttccattct 2480

ttaacagccc atcttggctt ggggttgcaa tgatggctgg gccagtcact tgtggcaggg 2540

catcagggcc ctggcaggga agaacctagg cacctggggt tgtccccagc ctgcccgtca 2600

gcatgagata cccagtggga aagtgagagg atgcggagag gttggcagag ccaggggtag 2660

gttctggagg ctcaagcaac aaggaggtgc aggtaaaggg tgcagtgcag ccactgaggg 2720

acagctggga actgggggat gcaagtgaga aagggatgtg ggggagagtt caggatcagg 2780

ctgcttgagg agtaatgggt tacctacagc agagacgaga tggctgtttg tcaggaggcg 2840

gtagaaaggt agaaaggatt cagattgtgg aagggtaaat ggatgagatg accattaagg 2900

ttttgtttta ctgagaggct gtaagtctgc cagggacagc tgtcagtaag gctgcagaag 2960

ggctgggggg ctacacaagg aagagcagaa ctagggttgt aagctcaaat gacgagcaaa 3020

cggtgagaag gaggcctgaa gggctgtgtg gggactatgg ctacctgaag agggcacacc 3080

ctgttaaagg ggccacagct gctcagctgt tcttacagtg ccggccctgt gttgccagat 3140
tgtctgcttt gtcagaggcc agaattctga ctttttatgt gaaataggat ttttaaatgc 3200
tggtgatcac ttaaaaaaaa cttaaaaacc caatactggc aaaagaggat gccagtttgc 3260
aatccctgaa gtagagagag ctcgtgctgg ggagaagtcc accaagatgc tttgaggcgg 3320
gctgtagcaa agtcctgttt ctcagagctg ggctgggctg gggtaggatc cttgcagctg 3380
aggaggagga aaagccactg agtctcctcc cagagcggga caaaccagag gccctttgca 3440
gttgctgggt caccagcggg ggtggcgcat ggagtacaga cagtgtagct cttggcctgc 3500
cagggagacg gatggtgcct ttgaagcaaa gaggaaggga aggcagaacc agggatgcct 3560
ttgctgatga gagtgcctgt cagggaaggc gccacaggct ggcagctctt caaaccagca 3620
gcgcttgacc cagagccaga tccagcatgg cctggccaga ggcaccctgg gaggccagct 3680
ggtcagtccc ttgcctcccc aagttccccc tggggtcaat gagccctggg aggatgccta 3740
acctaactcc agccagacta ataggggcat ggtgaccctt gactcaccat cccatcccag 3800
ctttcaggga gtgggggtag tgtggtctcc atgttcctac tatgcctaag aagagatggc 3860
tcaccttggg aggtgccagg ctgaaactag gtcctttccg ggtctggatg ctgccgctca 3920
ggagcagggg cgtggcctca gctgcctctg ggagcttccc gggaatgaca gggtttgagg 3980
ggagtagata tgagagggag ccgctcctgg ttctggagtc ttaggaggtt ccaacttgca 4040
ggatcctttc ccagagccct ccatggagaa aacagcaaaa tgaagccctt acctgcttgc 4100
tgtctgcaag ggagggagcc gagccccagc tgataatccc ccagcactca cccttcctga 4160
gctgagactt cggggctgtg gagaccagca caggacatag tggtgctttt taaatttatt 4220
tttaactgtt tctcatatgt agcaacccct cctcccctcc tgggcatgtt tacacaggct 4280
ctgctctggg ggctggcctg gctgtgaggt ttctggggag gcagagaggc agggactttg 4340
gggccttagt caccatccat ggtatcacct catctcactt cctgtgaggg acagggcctg 4400
gctgatgtga tcccagctcc ccccagttca ggactgtctt tcagctcctt tgcccctgga 4460
ggtgggggct gctggctgag gaggggtcaa ggtgagttca agaaagctac ctgtggaaaa 4520

tggaccaggt tgggggggtg attgcaaagt ctccccaaag cctggctcct catgctcagt 4580

gccaggggca gaacactggg gagccaggta tagagagcct tcctgtcata actgccagtc 4640

ctcttcctcc aaggcctctg catattctca tgttcccctc acccatcatg ccagccaccc 4700

ctatccctct tctagcaggg ccaagatggg gacagcagca gccctctggc cttgggatgt 4760

gatgataaag caagcctagg gccagggttt ggggagcaga gagagccaag aagttgacca 4820

cgtgtgattt ccagcccttc ccactgggac ttgacttccc aggtcaagga gtccgtctca 4880

ttctggctgg tcgagtgacc agaggcctgt gtgaatgtgt gcacctgctt ttcctgcctg 4940

gaatgttttc tggctcagct gcagcaacat ctgtgagccc agtgtctgcc ctgtgtccct 5000

gggctcgctc caagtgcagg aacatacatg cagggcccaa catgatgatg gtgtgaaggg 5060

caggaaacag tcctctgaag gagtggggag gtgggcagtc tgcccccgcc aggtaccatc 5120

gcctcctgcc agcttcctta gaccaggcag ggctgccatg gtgctagctg caagtccatc 5180

agtattgacc gtctcgctcc atcttggtcc tccggagtcc caagtttcct tttcatcaaa 5240

tctgacaaga gagaagaaac atgggtgtgc ttggcccaca gggcctggtg gtgatggacc 5300

tccccgctcc ctcaagctct ggatggctgc agtgttgtac tagactttgt tcaggctgtt 5360

ctcatctcag tattgcccct tcctttcact ttcacacttc atctcattcc tgttgtcact 5420

ttccccgaaa cgaataaagt ctccccagct cctgctgtgt aggctgggca gaaaccacaa 5480

c 5481

<210> 2
<211> 365
<212> PRT
<213> Homo sapiens

<400> 2
Met Pro Gly Gly Lys Lys Val Ala Gly Gly Gly Ser Ser Gly Ala Thr
1               5                   10                  15

Pro Thr Ser Ala Ala Ala Thr Ala Pro Ser Gly Val Arg Arg Leu Glu
            20                  25                  30

Thr Ser Glu Gly Thr Ser Ala Gln Arg Asp Glu Glu Pro Glu Glu Glu
        35                  40                  45

Gly Glu Glu Asp Leu Arg Asp Gly Gly Val Pro Phe Phe Val Asn Arg
    50                  55                  60

Gly Gly Leu Pro Val Asp Glu Ala Thr Trp Glu Arg Met Trp Lys His
65                  70                  75                  80

Val Ala Lys Ile His Pro Asp Gly Glu Lys Val Ala Gln Arg Ile Arg
                85                  90                  95

Gly Ala Thr Asp Leu Pro Lys Ile Pro Ile Pro Ser Val Pro Thr Phe
            100                 105                 110

Gln Pro Ser Thr Pro Val Pro Glu Arg Leu Glu Ala Val Gln Arg Tyr
        115                 120                 125

Ile Arg Glu Leu Gln Tyr Asn His Thr Gly Thr Gln Phe Phe Glu Ile
    130                 135                 140

Lys Lys Ser Arg Pro Leu Thr Gly Leu Met Asp Leu Ala Lys Glu Met
145                 150                 155                 160

Thr Lys Glu Ala Leu Pro Ile Lys Cys Leu Glu Ala Val Ile Leu Gly
                165                 170                 175

Ile Tyr Leu Thr Asn Ser Met Pro Thr Leu Glu Arg Phe Pro Ile Ser
            180                 185                 190

Phe Lys Thr Tyr Phe Ser Gly Asn Tyr Phe Arg His Ile Val Leu Gly
        195                 200                 205

Val Asn Phe Ala Gly Arg Tyr Gly Ala Leu Gly Met Ser Arg Arg Glu
    210                 215                 220

Asp Leu Met Tyr Lys Pro Pro Ala Phe Arg Thr Leu Ser Glu Leu Val
225                 230                 235                 240

Leu Asp Phe Glu Ala Ala Tyr Gly Arg Cys Trp His Val Leu Lys Lys
                245                 250                 255

Val Lys Leu Gly Gln Ser Val Ser His Asp Pro His Ser Val Glu Gln
            260                 265                 270

Ile Glu Trp Lys His Ser Val Leu Asp Val Glu Arg Leu Gly Arg Asp
        275                 280                 285

Asp Phe Arg Lys Glu Leu Glu Arg His Ala Arg Asp Met Arg Leu Lys
    290                 295                 300

Ile Gly Lys Gly Thr Gly Pro Pro Ser Pro Thr Lys Asp Arg Lys Lys
305                 310                 315                 320

Asp Val Ser Ser Pro Gln Arg Ala Gln Ser Ser Pro His Arg Arg Asn
                325                 330                 335

Ser Arg Ser Glu Arg Arg Pro Ser Gly Asp Lys Lys Thr Ser Glu Pro
            340                 345                 350

Lys Ala Met Pro Asp Leu Asn Gly Tyr Gln Ile Arg Val
        355                 360                 365

<210>  3
<211>  1068
<212>  DNA
<213>  Homo sapiens

<400>  3
atgaccggct ccgcggccga cactcaccgc tgcccccacc ccaaaggcgc caaaggcacc      60

cggtcccgga gcagccacgc gcggcccgtg agcctcgcca ccagcggggg ctcagaggag     120

gaggacaaag acggcggggt gctgttccac gtcaacaaga gcggcttccc catcgacagc     180

cacacctggg agcgcatgtg gatgcacgtg gccaaggtgc accctaaggg gggagaaatg     240

gtgggcgcca tcaggaacgc cgccttcttg gcaaagcctt caatacccca ggtcccaaac     300

tacaggctgt cgatgacgat cccagactgg ctccaggcga tccagaatta catgaagacc     360

ctacaatata atcacacagg gacccagttc tttgaaatta ggaaaatgag accgctgagt     420

gggttaatgg aaacagcaaa agaaatgacc cgagagtcct tgcctatcaa atgccttgaa     480

gctgtcatcc tgggcatcta cttaaccaat gggcagcctt ccattgagcg gttccccatc     540

agctttaaaa cctacttctc aggaaactac tttcaccacg ttgtgctggg gatttactgc     600

aatggccgct atggctcatt gggcatgagc cgcagggctg agctgatgga caagccattg     660

acttttcgga ctctgagtga cctcatcttt gactttgagg actcttacaa gaaatacctg     720

cacacagtca agaaggtcaa gattgggctg tacgtccccc atgagcctca tagcttccag     780

cccattgagt ggaagcagct ggtcctcaac gtctcaaaga tgctgagggc tgacataagg 840

aaggagctgg agaaatatgc cagggacatg agaatgaaga tcctgaaacc tgcaagtgcc 900

cactctccga cccaagtgag aagccgggga aaatccctgt cccccagaag gagacaggca 960

agccccccga ggaggctcgg ccggcgagag aagtcgcctg cactgcctga aaagaaggtg 1020

gctgatctga gcactctgaa tgaagtgggc tatcaaatcc gaatttag 1068

<210> 4
<211> 355
<212> PRT
<213> Homo sapiens

<400> 4
Met Thr Gly Ser Ala Ala Asp Thr His Arg Cys Pro His Pro Lys Gly
1               5                   10                  15

Ala Lys Gly Thr Arg Ser Arg Ser Ser His Ala Arg Pro Val Ser Leu
            20                  25                  30

Ala Thr Ser Gly Gly Ser Glu Glu Glu Asp Lys Asp Gly Gly Val Leu
        35                  40                  45

Phe His Val Asn Lys Ser Gly Phe Pro Ile Asp Ser His Thr Trp Glu
    50                  55                  60

Arg Met Trp Met His Val Ala Lys Val His Pro Lys Gly Gly Glu Met
65                  70                  75                  80

Val Gly Ala Ile Arg Asn Ala Ala Phe Leu Ala Lys Pro Ser Ile Pro
                85                  90                  95

Gln Val Pro Asn Tyr Arg Leu Ser Met Thr Ile Pro Asp Trp Leu Gln
            100                 105                 110

Ala Ile Gln Asn Tyr Met Lys Thr Leu Gln Tyr Asn His Thr Gly Thr
        115                 120                 125

Gln Phe Phe Glu Ile Arg Lys Met Arg Pro Leu Ser Gly Leu Met Glu
    130                 135                 140

Thr Ala Lys Glu Met Thr Arg Glu Ser Leu Pro Ile Lys Cys Leu Glu
145                 150                 155                 160

Ala Val Ile Leu Gly Ile Tyr Leu Thr Asn Gly Gln Pro Ser Ile Glu

                165                 170                 175

Arg Phe Pro Ile Ser Phe Lys Thr Tyr Phe Ser Gly Asn Tyr Phe His
            180                 185                 190

His Val Val Leu Gly Ile Tyr Cys Asn Gly Arg Tyr Gly Ser Leu Gly
        195                 200                 205

Met Ser Arg Arg Ala Glu Leu Met Asp Lys Pro Leu Thr Phe Arg Thr
    210                 215                 220

Leu Ser Asp Leu Ile Phe Asp Phe Glu Asp Ser Tyr Lys Lys Tyr Leu
225                 230                 235                 240

His Thr Val Lys Lys Val Lys Ile Gly Leu Tyr Val Pro His Glu Pro
                245                 250                 255

His Ser Phe Gln Pro Ile Glu Trp Lys Gln Leu Val Leu Asn Val Ser
            260                 265                 270

Lys Met Leu Arg Ala Asp Ile Arg Lys Glu Leu Glu Lys Tyr Ala Arg
        275                 280                 285

Asp Met Arg Met Lys Ile Leu Lys Pro Ala Ser Ala His Ser Pro Thr
    290                 295                 300

Gln Val Arg Ser Arg Gly Lys Ser Leu Ser Pro Arg Arg Arg Gln Ala
305                 310                 315                 320

Ser Pro Pro Arg Arg Leu Gly Arg Arg Glu Lys Ser Pro Ala Leu Pro
                325                 330                 335

Glu Lys Lys Val Ala Asp Leu Ser Thr Leu Asn Glu Val Gly Tyr Gln
            340                 345                 350

Ile Arg Ile
        355